(19) Europäisches Patentamt
European Patent Office
Office européen des brevets

(11) EP 2 873 663 A1

# (12) EUROPEAN PATENT APPLICATION

(43) Date of publication:
**20.05.2015 Bulletin 2015/21**

(21) Application number: **13306584.7**

(22) Date of filing: **19.11.2013**

(51) Int Cl.:
*C07D 401/12* (2006.01) *A61K 31/473* (2006.01)
*A61P 25/00* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicants:
- **Université de Strasbourg**
  **67000 Strasbourg (FR)**
- **COMMISSARIAT A L'ENERGIE ATOMIQUE ET AUX ENERGIES ALTERNATIVES**
  **75015 Paris (FR)**
- **Centre National de la Recherche Scientifique**
  **75016 Paris (FR)**
- **UNIVERSITE DE ROUEN**
  **76130 Mont-Saint-Aignan (FR)**
- **L'ETAT FRANCAIS (MINISTERE DE LA DEFENSE) représenté par le Directeur de l'Institut de Recherche Biomédicale des Armées**
  **91224 Bretigny Sur Orge (FR)**

(72) Inventors:
- **Baati, Rachid**
  **67460 SOUFFELWEYERSHEIM (FR)**
- **Kliachyna, Maria**
  **67400 ILLKIRCH GRAFFENSTADEN (FR)**
- **Nussbaum, Valentin**
  **68390 SAUSHEIM (FR)**
- **Renard, Pierre-Yves**
  **75004 PARIS (FR)**
- **Jean, Ludovic**
  **76000 ROUEN (FR)**
- **Weik, Martin**
  **38120 SAINT EGREVE (FR)**
- **Nachon, Florian**
  **38400 GIERES (FR)**
- **Touvrey, Mélanie**
  **38420 LE VERSOUD (FR)**
- **Friedel, Mélanie**
  **38100 GRENOBLE (FR)**
- **Renou, Julien**
  **76170 LA FRENAYE (FR)**
- **Verdelet, Tristan**
  **76000 ROUEN (FR)**
- **Mercey, Guillaume**
  **27400 MONTAURE (FR)**
- **Colletier, Jacques-Philippe**
  **38300 BOURGOIN JALLIEU (FR)**
- **Sanson, Benoît**
  **NEW YORK, NY New York 11776 (US)**
- **Santoni, Gianluca**
  **38000 GRENOBLE (FR)**

(74) Representative: **Tezier Herman, Béatrice et al**
**Cabinet Becker & Associés**
**25, rue Louis le Grand**
**75002 Paris (FR)**

## (54) Novel uncharged reactivators against OP-inhibition of human acetylcholinesterase

(57) The present invention deals with novel uncharged reactivators of human acetylcholinesterase of formula (II) below, pharmaceutical compositions comprising said compounds, and their use for reactivating human acetylcholinesterase inhibited by at least one organophosphorus nerve agent.

EP 2 873 663 A1

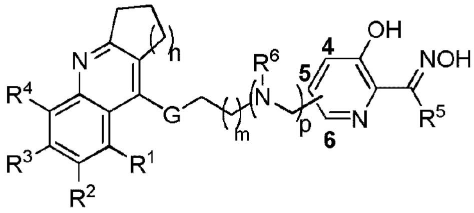

(II)

**Description**

## Introduction

The present invention deals with novel uncharged reactivators of human acetylcholinesterase, pharmaceutical compositions comprising said compounds, and their use for reactivating human acetylcholinesterase inhibited by at least one organophosphorus nerve agent.

## Background of the invention

Organophosphorus nerve agents (OPNA) are extremely toxic compounds that include chemical warfare agents (CWA) (sarin, soman, cyclosarin, tabun, methylphosphonothioate VX) and pesticides (paraoxon, parathion, tetraethyl pyrophosphate (TEPP)). Their acute toxicity results from the irreversible inhibition of acetylcholinesterase (AChE) through phosphylation of its catalytic serine. Accumulation of neurotransmitter acetylcholine (ACh) at cholinergic synapses ensues, leading to nervous and respiratory failures. Depending on the class of OPNA and on the administrated dose, death can occur within minutes.
Due to the similarity between the chemical precursors of CWA and pesticides, and to the relatively simple chemistry involved in their synthesis, efforts to control the proliferation of these agents have proved of limited success. Illustrative examples include the terrorist attack in the Tokyo subway in 1995, the bombing of Kurd civilians during the Iraq-Iran war in 1988, and that of civilians in Syria, as reported in August 2013. Additionally, despite the international efforts aimed at regulating and lessening the use of these environmentally toxic compounds, ca. 100 different OPNA are still used intensively as pest control agents, with only anecdotal monitoring. This results in about 3,000,000 acute intoxications per year, 200,000 of which lead to death. Therefore, the development of effective measures to counteract organophosphorus (OP) poisoning remains a challenging issue to protect and treat both civilian and military populations. The current treatment against OP poisoning consists in the administration of a combination of atropine (antimuscarinic agent) and diazepam (anticonvulsant drug), to limit convulsions, and of a standard pyridinium oxime (pralidoxime, trimedoxime, HI-6, obidoxime, or HLö-7) to reactivate AChE. Oximes exert their action on OP-inhibited AChE by attacking the phosphorus atom of the phosphylated serine, yielding to the removal of the phosphonate and recovery of the enzyme's catalytic activity. To this end, pyridinium oximes must display high nucleophilicity, which is generally attained by the formation of an oximate anion at physiological pH. As of today, however, not a single oxime has proven equally effective against all types of OP-inhibited AChE, and most are ineffective against tabun-inhibited hAChE.
Another weakness of currently approved pyridinium aldoximes is their difficulty in crossing the blood-brain barrier (BBB) owing to the permanent positive charge carried by the pyridinium nitrogen atom. For example, it was estimated using *in vivo* rat brain microdialysis coupled with HPLC/UV, that the BBB penetration of the most commonly used oxime, 2-PAM, is only 10%. Therefore, oximes reactivating AChE in the peripheral nervous system are not effective in the brain and, consequently, do not protect against the neurological effects of OP-exposure.
Neutral oxime-based compounds, both able to cross the BBB and to reactivate OP-inhibited AChE in the Central Nervous System, have also been synthesized.
Coupling of an oxime-based structure to a potential ligand of the peripheral site of the enzyme (PSL) was also investigated to increase affinity of the reactivator for AChE.
AChE reactivators obtained by these strategies are often less efficient than currently used pyridinium oximes. Further, these compounds are often efficient for only some types of OP-inhibited-AChE. Finally, these compounds sometimes present low affinity for the enzyme, and consequently high concentrations must be used for obtaining the desired reactivation.
In particular, tabun-hAChE is known to be reluctant to reactivation due to weak eletrophilicity and steric hindrance of the tabun-hAChE adduct. None of the previously described reactivating compounds obtained by any of the above mentioned strategies is able to reactivate all types of inhibited hAChE, in particular tabun-hAChE.
The choice of the peripheral site ligands that can be coupled to the oxime moiety to create bifunctional reactivators with enhanced affinity to hAChE as disclosed above is based on their character of inhibitor of native hAChE.
In this context, the Applicant surprisingly found that bifunctional compounds comprising a peripheral site ligand moiety with a tacrine (1,2,3,4-tetrahydroacridine) structure, a specific linker, and an oxime moiety are potent reactivators of human AChE inhibited with any type of organophosphorus compounds. Tacrines are however known to be not among the best inhibitors of hAChE.

## Summary of the invention

A first object of the present invention is a compound of formula (I)

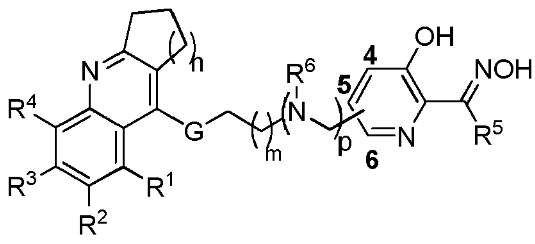

(I) ,

wherein
the different groups are as defined in the detailed description below.
Another object of the present invention is a pharmaceutical composition comprising at least one compound of formula (I) and at least one pharmaceutically acceptable support. Another object of the invention is a compound or a composition according to the invention, for use as a medicament.
Another object of the invention is a compound or a composition according to the invention, for use as a reactivator of human acetylcholinesterase *in vitro* and/or *in vivo,* wherein the human acetylcholinesterase was inhibited by at least one organophosphorus nerve agent.
A last object of the invention is a compound or a composition according to the invention for use in the treatment of a nervous and/or respiratory failure due to intoxication with at least one organophosphorus nerve agent.

**Brief description of the drawings:**

Figure 1 : Docked conformation of molecule **12** at the peripheral site of VX-hAChE.
Figure 2: Distribution of distances between the aldoxime oxygen and the VX phosphorus analyzed along the simulation trajectory for the four different linker lengths (KM 2 : $-CH_2-CH_2-$; KM 3: $-CH_2-CH_2-CH_2-$ , KM 4: $-CH_2-CH_2-CH_2-CH_2-$, 12; KM 5: $-CH_2-CH_2-CH_2-CH_2-CH_2-$, 11).

**Detailed description of the invention**

A first object of the invention is a compound of formula (I):

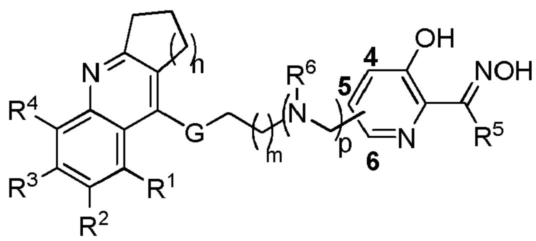

(I) ,

wherein

G is selected from the group consisting of $CH_2$, O, S, NH, NR, C(O)-NH, C(O)-NR, NH-C(O), NR-C(O), NH-C(S), NR-C(S), NH-NR, NR-NR', NH-O, NR-O, NH-C-(O)-NH, NR-C(O)-NH, NR-C(O)-NR', NH-C(S)-NH, NR-C(S)-NH, and NR-C(S)-NR', preferably G is NH,
wherein each R or R' is independently selected from the group consisting of a hydrogen atom, an alkyl group, an acyl group, an aryl group and a heteroaryl group;
n is 1, 2, 3 or 4, preferably n is 2,
m is 0, 1, 2, 3, or 4, preferably m is 3,
p is 0 or 1, preferably p is 0,
$R^1$, $R^2$, $R^3$ and $R^4$ are independently selected from the group consisting of a hydrogen atom, a halogen atom, an alkyl group, an alkoxy group, an amine NHR" group, an amide NR"-C(O)-R"' group, an oligopeptide or a polyethyleneglycol (PEG) chain, such as a 2-hydroxyethoxy group ($OCH_2CH_2OH$), wherein R" and R"' have the same definition as R and $R^1$,
$R^5$ is a hydrogen atom, a trifluoromethyl ($CF_3$) group, or a $NH_2$ group, preferably $R^5$ is a hydrogen atom, and
$R^6$ is a hydrogen atom, an alkyl group, an aryl group or an acyl group, preferably $R^6$ is an alkyl group, in particular a methyl group.

In an embodiment, the pyridine is substituted in position 6.
In an embodiment, three among $R^1$, $R^2$, $R^3$ and $R^4$ are hydrogen atoms. In another embodiment, $R^1$, $R^2$, $R^3$ and $R^4$ are hydrogen atoms.
In the present invention, an "alkyl group" is a linear, branched or cyclic saturated hydrocarbon group comprising from 1 to 10 carbon atoms, preferably from 1 to 6 carbon atoms, in particular from 1 to 3 carbon atoms. As examples of alkyl groups may be cited the methyl, ethyl, n-propyl, isopropyl, n-butyl, isobutyl, tert-butyl, n-pentyl, isopentyl, n-hexyl, and cyclohexyl groups.
An "alkoxy group" is an alkyl group, linked to the rest of the compound *via* an oxygen atom (ether linkage).
An "aryl group" refers to a monocyclic or polycyclic hydrocarbon aromatic group, with at least one of the rings having a system of conjugated pi electrons, which may be optionally substituted. Examples of aryl groups that can be used are optionally substituted phenyl, naphthalene and anthracene. Preferably, the aryl group is a phenyl group, optionally susbtituted.
A "heteroaryl group" is an aryl group, wherein at least one carbon atom is replaced with a heteroatom such as N, O, P or S. Examples of heteroaryl groups include pyrrole, thiophene, furane, pryridine, pyrimidine, pyrazine, triazine, imidazole, thiazole, oxazole, and isoxazole.
An "acyl group" is an alkyl group or an aryl group, linked to the rest of the compound *via* a C(=O) linkage.
A "halogen atom" is an atom of F, Cl, Br or I. An "oligopeptide" is a polymer of 2 to 10 amino acids, linked to each other *via* peptidic bonds. Preferably, an oligopeptide according to the invention comprises from 2 to 6 amino acids. Examples of amino acids include alanine, arginine, asparagine, aspartate, cysteine, glutamate, glutamine, glycine, histidine, isoleucine, leucine, lysine, methionine, phenylalanine, proline, pyrrolysine, selenocysteine, serine, threonine, tryptophane, tyrosine and valine. Amino acids are preferably L-amino acids.
A "polyethyleneglycol chain" is a group of the formula $[O-(CH2)_2]_a$-OH, wherein a is an integer from 1 to 150, preferably from 1 to 10.
An "organophosphorus nerve agent" (OPNA) is any organophopshorus compound having an effect on the nervous system. This term designates for instance chemical warfare agents, such as sarin, soman, cyclosarin, tabun, methylphosphonothioate, VX (*O*-ethyl *S*-[2-(diisopropylamino)ethyl] methylphosphonothioate) and pesticides such as paraoxon, parathion and tetraethyl pyrophosphate.
The compounds of the invention are preferably selected from:

N',3-dihydroxy-4-{5-[(1,2,3,4-tetrahydroacridin-9-yl)amino]pentyl}pyridine-2-carboximidamide (6)
N',3-dihydroxy-6-{5-[(1,2,3,4-tetrahydroacridin-9-yl)amino]pentyl}pyridine-2-carboximidamide (7)
(Z)-N',3-dihydroxy-4-{[methyl({2-[(1,2,3,4-tetrahydroacridin-9-yl)amino]ethyl}) amino]methyl}pyridine-2-carboximidamide **(8)**
(Z)-N',3-dihydroxy-4-{[methyl({3-[(1,2,3,4-tetrahydroacridin-9-yl)amino]propyl}) amino]methyl}pyridine-2-carboximidamide **(9),**
2-[(hydroxyimino)methyl]-4- {5-[(1,2,3,4-tetrahydroacridin-9-yl)amino]pentyl} pyridin-3-ol(**10**),
2-[(1E)-(hydroxyimino)methyl]-6-{5-[(1,2,3,4-tetrahydroacridin-9-yl)amino]pentyl} pyridin-3-ol (**11**)
2-[1-(hydroxyimino)methyl]-6-{4-[(1,2,3,4-tetrahydroacridin-9-yl)amino]butyl} pyridin-3-ol **(12),**
2-[1-(hydroxyimino)methyl]-5-{5-[(1,2,3,4-tetrahydroacridin-9-yl)amino]pentyl} pyridin-3-ol **(13),** and
2-[1-(hydroxyimino)methyl]-6-[5-(1,2,3,4-tetrahydroacridin-9-ylsulfanyl)pentyl] pyridin-3-ol **(14).**

In an embodiment, the compounds of the invention are selected among compounds **6, 7, 8, 9, 10, 11, 12** and **13.** In a preferred embodiment, the compounds of the invention are selected among compounds **11** and **12.** In a highly preferred embodiment, the compound of the invention is compound **12.**

*Synthesis of the compounds of the invention*

A compound of formula (I) according to the invention may be synthesized by any appropriate method known by anyone of ordinary skill in the art.
In particular, the process for the synthesis of compounds of formula (I) may comprise a step of Sonogashira coupling reaction between a halogenated pyridine derivative and a compound comprising a terminal alkyne and a tacrine moiety. The resulting alkyne may be reduced by reaction with hydrogen, for instance in presence of Pearlmann's catalyst

$Pd(OH)_2/C$.

For the compounds of formula (I) with $R^5$ = H, the oxime may be formed from the corresponding aldehyde by reaction with $NH_2OH$, preferably as a hydrochloride, preferably in presence of a base, such as $CH_3COONa$.

For the compounds of formula (I) with $R^5$ = $NH_2$, the amidoxime may be formed from the corresponding nitrile by reaction with $NH_2OH$, preferably as a hydrochloride, preferably in presence of a base, such as pyridine.

*Pharmaceutical use of the compounds of the invention*

Another object of the invention is a pharmaceutical composition comprising at least one compound according to the invention and a pharmaceutically acceptable support.

The pharmaceutical composition comprising the compound of formula (I) according to the invention can be prepared by mixing the compound with a physiologically acceptable support, an excipient, a binder and/or a diluent.

The amount of compound of formula (I) in the composition according to the invention may evolve in a large scope depending upon the patient, the mode of administration and the expected effect.

The compound or composition according to the invention can be administered orally or non-orally, for instance *via* parenteral, intramuscular, intravenous, cutaneous, nasal or rectal route.

The pharmaceutical composition of the invention can present different forms including granules, powders, tablets, gel capsules, syrups, emulsions, suspensions, and forms used for non-oral administration, for instance injections, sprays or suppositories. These pharmaceutical forms can be prepared *via* known conventional techniques.

The preparation of an orally administered solid pharmaceutical form can be for instance performed by the following process: an excipient (for example lactose, sucrose, starch or mannitol), a disintegrant (for example calcium carbonate, calcium carboxymethylcellulose, alginic acid, sodium carboxymethylcellulose, colloidal silicon dioxide, sodium croscarmellose, Crospovidone, guar gum, magnesium aluminium silicate, microcrystalline cellulose, cellulose powder, pregelatinised starch, sodium alginate or starch glycolate), a binder (for example alpha-starch, gum arabic, carboxymethylcellulose, polyvinylpyrrolidone, hydroxypropylcellulose, alginic acid, carbomer, dextrin, ethylcellulose, sodium alginate, maltodextrin, liquid glucose, magnesium aluminium silicate, hydroxyethylcellulose, methylcellulose or guar gum) and a lubricant (for example talc, magnesium stearate or polyethylene 6000) are added to the active principle and the mixture obtained is then tabletted. If necessary, the tablet can be coated via the known techniques, in order to mask the taste (for example with cocoa powder, mint, borneol or cinnamon powder) or to allow enteric dissolution or sustained release of the active principles. Coating products that can be used are, for example, ethylcellulose, hydroxymethylcellulose, polyoxyethylene glycol, cellulose acetophthalate, hydroxypropylmethylcellulose phthalate and Eudragit® (methacrylic acid-acrylic acid copolymer), Opadry® (hydroxypropylmethylcellulose + macrogol + titanium oxide + lactose monohydrate). Pharmaceutically acceptable colorants may be added (for example yellow iron oxide, red iron oxide or quinoline yellow lake).

Liquid pharmaceutical forms for oral administration include solutions, suspensions and emulsions. The aqueous solutions can be obtained by dissolving the active principle in water, followed by addition of flavourings, colorants, stabilisers and/or thickeners, if necessary. In order to improve the solubility, it is possible to add ethanol, propylene glycol or any other pharmaceutically acceptable non-aqueous solvent. The aqueous suspensions for oral use can be obtained by dispersing the finely divided active principle in water with a viscous product, such as a natural or synthetic gum or resin, methylcellulose or sodium carboxymethylcellulose.

The pharmaceutical forms for injection can be obtained, for example, by the following process. The active principle is dissolved, suspended or emulsified either in an aqueous medium (for example distilled water, physiological saline or Ringer's solution) or in an oily medium (for example olive oil, sesame seed oil, cottonseed oil, corn oil or propylene glycol), with a dispersant (for example Tween® 80, HCO® 60 (Nikko Chemicals), polyethylene glycol, carboxymethylcellulose or sodium alginate), a preserving agent (for example methyl p-hydroxybenzoate, propyl p-hydroxybenzoate, benzyl alcohol, chlorobutanol or phenol), an isotonicity agent (for example sodium chloride, glycerol, sorbitol or glucose) and optionally other additives, such as, if desired, a solubilising agent (for example sodium salicylate or sodium acetate) or a stabiliser (for example human serum albumin).

Pharmaceutical forms for external use can be obtained from a solid, semi-solid or liquid composition containing the active principle. For example, to obtain a solid form, the active principle can be treated with excipients (for example lactose, mannitol, starch, microcrystalline cellulose or sucrose) and a thickener (for example natural gums, cellulose derivatives or acrylic polymers) so as to convert them into powder. The liquid pharmaceutical compositions are prepared in substantially the same way as the forms for injection, as indicated previously. The semi-solid pharmaceutical forms are preferably in the form of aqueous or oily gels or in the form of pomades. These compositions may optionally contain a pH regulator (for example carbonic acid, phosphoric acid, citric acid, hydrochloric acid or sodium hydroxide) and a preserving agent (for example a p-hydroxybenzoic acid ester, chlorobutanol or benzalkonium chloride).

Another object of the invention is a compound according to the invention, for use as a medicament.

Another object of the invention is a compound according to the invention, for *in vivo* and/or *in vitro* use as a reactivator

of human acetylcholinesterase, wherein the human acetylcholinesterase was inhibited by at least one organophosphorus nerve agent. Another object of the present invention is a compound according to the invention, for use in the treatment of a disorder casued by at least one organosphosphorus nerve agent. Another object of the present invention is a compound according to the invention, for use in the treatment of a nervous and/or respiratory failure due to intoxication with at least one organophosphorus nerve agent.

Another object of the present invention is a method for the treatment of a nervous and/or respiratory failure due to intoxication with at least one organophosphorus nerve agent, comprising administering at least one compound according to the invention. Another object of the present invention is the use of a compound according to the invention for the preparation of a medicament for the treatment of a nervous and/or respiratory failure due to intoxication with at least one organophosphorus nerve agent.

Within the context of the invention, the term treatment denotes curative, symptomatic, and/or preventive treatments. In particular, it can refer to reducing the progression of the disease, reducing or suppressing at least one of its symptoms or complications, or improving in any way the state of health of patients.

The administration of the compounds or of the composition according to the invention may be performed either before or after the exposition of the subject to the organophosphorus nerve agent. The term "after" also includes the possibility for the administration and the exposition to the OP to be simultaneous.

In the present invention, the terms "subject" and "patient" are used indifferently and designate a human subject.

The amount of compound of formula (I) to be administered according to the invention may evolve in a large scope depending upon the patient, the mode of administration and the expected effect. In particular, the amount of compound of formula (I) may be comprised between 200 mg and 4000 mg, with up to 3 takes per day.

The compound or composition according to the invention may be co-administered with at least one other active agent, such as an antimuscarinic agent, in particular atropine, an anticonvulsant, in particular diazepam or one of its prodrugs, such as avizafone, and/or a bioscavenger able to capture and/or degrade OPs in blood, such as human butyrylcho linesterase.

The term co-administered means that the administration of the compound or composition according to the invention and that of the other active agent can be simultaneous, sequential and /or separate.

The following examples are provided as illustrative, and not limitative, of the present invention.

## Examples:

Example 1 : Synthesis of compounds of the invention

***General procedure 1*** for the Sonogashira coupling reaction (synthesis of compounds **20, 23, 35, 36,** and **37).** The flask containing solution of iodo- or bromopyridine (1 equiv) in THF/$Et_3N$ (tetrahydrofuran/triethylamine) was evacuated and filled with argon three times before the addition of catalysts $Pd(PPh_3)_4$ (0.1 equiv) and CuI (0.2 equiv). After degazation with Argon the mixture was stirred at the room temperature for 5 min, then the degazed solution of alkyne (1.1 equiv) was added and the reaction mixture was stirred during 20 h at the room temperature. After concentration at reduced pressure the residue was purified by the column chromatography.

***General procedure* 2** for *N*-(diethylamino)carbamate group removal and nitrile conversion to amidoxime (synthesis of compounds **6, 8,** and **9).** The solution of **21, 28** or 29 (1 equiv), $NH_2OH.HCl$ (30 equiv), and pyridine (30 equiv) in 2 mL of ethanol was refluxed during 14 h. The solvent was removed in *vacuum,* the residue was subjected to preparative HPLC chromatography.

***General procedure* 3** for the one-pot alkyne reduction and O-debenzylation (synthesis of compounds **24, 38, 39,** and **40).** To the degazed solution of alkyne (1 equiv) in ethyl acetate or methanol Pearlman's catalyst $Pd(OH)_2$/C (20%, moisture 50%, 0.3 equiv) was added. After the degazation the solution was bubbled with $H_2$. The reaction mixture was stirred at RT (room temperature) under $H_2$ (1 atm) during 24 h. The solution was filtered through Celite®, the solvent was evaporated, and the product was dried in *vacuum.*

***General procedure 4*** for the three-step transformation of the methyl ester into aldehyde (synthesis of compounds **31, 41, 42, 43** and **50).** The solution of methyl ester **30, 38, 39, 40** or **49** (1 equiv), imidazole (4.15 equiv), and TBDMSCl (tert-Butyldimethylsilyl chloride)(2.4 equiv) in dry DMF (dimethylformamide) was stirred at room temperature under argon during 2 h. Ethyl acetate was added, and the organic phase was washed with water 3 times, dried over $Na_2SO_4$ and concentrated in *vacuum.* After drying in *vacuum,* the compound obtained was subjected without purification to the following reduction of methyl ester with DIBAL-H (diisobutylaluminium hydride). To the solution of the compound obtained (1 equiv) in dry $CH_2Cl_2$ at -78 °C DIBAL-H (1M solution in $CH_2Cl_2$, 2 equiv) was added dropwise. The reaction mixture was stirred at-78 °C for 12 min, then it was quenched with MeOH, and the cooling bath was removed. When the mixture was warmed to the room temperature, the organic phase was washed with aqueous solution of NaOH (1M), dried over $Na_2SO_4$ and concentrated under reduced pressure. After drying in *vacuum* the product obtained was subjected to the following reaction with TBAF (tetra n-butylammonium fluoride). To the solution of the compound obtained (1 equiv) in

dry THF at 0 °C TBAF (1 M solution in THF, 1.1 equiv) was added, and the reaction mixture was stirred at this temperature for 1 h. After removal of the solvent under reduced pressure the residue was purified by column chromatography (silica gel).

***General procedure 5*** for synthesis of oximes **10, 11, 12, 13,** and **14.** The solution of aldehyde **31, 41, 42, 43** or **50** (1 equiv), $NH_2OH.HCl$ (1.5 equiv), and $CH_3COONa$ (1.5 equiv) in dry ethanol was stirred during 8 h. After concentration under reduced pressure the crude product was purified by the column chromatography or preparative HPLC.

**2-Cyanopyridin-3-yl N,N-diethylcarbamate (17)**

To the solution of 2-cyano-3-hydroxypyridine **16** (2.00 g, 0.01665 mol, 1 equiv) in 40 mL of pyridine at 0 °C diethylcarbamoylchloride (2.2 mL, 2.37 g, 0.01748 mol, 1.05 equiv) was added. The reaction mixture was stirred at the room temperature overnight. The solvent was removed in *vacuum.* The resulting crude material was treated with water. Then the mixture was neutralized with HCl, and the resulting product was extracted with ethyl ether, the extract was washed with aqueous 10% $Na_2CO_3$ and brine, dried over $Na_2SO_4$. The solvent was evaporated and product was dried in *vacuum.* The crude product was purified by column chromatography (silica gel, ethyl acetate/c-hexane = 1/2). $R_f$ = 0.45 (ethyl acetate/c-hexane = 2/3). Yield 3.46 g, 95%. $^1H$ NMR (400 MHz, $CDCl_3$, $\delta$): 1.21 (3H, t, J = 7.2 Hz, $CH_3$); 1.30 (3H, t, J = 7.2 Hz, $CH_3$); 3.38 (2H, q, J = 7.2 Hz, $CH_2$); 3.49 (2H, q, J = 7.2 Hz, $CH_2$); 7.49-7.52 (1H, dd, J = 8.5 Hz, J = 4.6 Hz, ArH); 7.82-7.84 (1H, dd, J = 8.5 Hz, J = 1.3 Hz, ArH); 8.47-8.49 (1H, dd, J = 4.6 Hz, J = 1.3 Hz, ArH). $^{13}C$ NMR (100 MHz, $CDCl_3$, $\delta$): 13.34; 14.31; 42.61; 43.02; 114.69; 127.50; 131.23; 147.17; 151.56; 151.97. HRMS (ESI): m/z: calcd for $C_{11}H_{14}N_3O_2$ $[M+H]^+$: 220.10860, found 220.10826.

**2-Cyano-4-iodopyridin-3-yl N,N-diethylcarbamate (18)**

To the solution of 2-cyano-pyridine-3-yl-diethylcarbamate 17 (1.00g, 0.00456 mol, 1 equiv) in 40 mL of THF n-BuLi solution (1.6M, in hexanes, 4.28 mL, 0.006841 mol, 1.5 equiv) was added dropwise at -78 °C. After 30 min of stirring at -78 °C 10 mL of $I_2$ solution in THF (0.006841 mol, 1.5 equiv) was added. The reaction mixture was stirred for 1 h at -78 °C, then 20 mL of aqueous $NH_4Cl$ solution (10%) was added. The organic phase was separated, the aqueous phase was extracted with ethyl ether, and the combined organic phases were washed with brine. After removal of the solvents the crude product was subjected to the column chromatography (silica gel, ethyl acetate/c-hexane = 1/5). $R_f$ = 0.47 (ethyl acetate/c-hexane = 1/3). Yield 48%. $^1H$ NMR (400 MHz, $CDCl_3$, $\delta$): 1.24 (3H, t, J = 7.2 Hz, $CH_3$); 1.36 (3H, t, J = 7.2 Hz, $CH_3$); 3.41 (2H, q, J = 7.2 Hz, $CH_2$); 3.53 (2H, q, J = 7.2 Hz, $CH_2$); 7.96 (1H, d, J = 5 Hz, ArH); 8.12 (1H, d, J = 5 Hz, ArH). $^{13}C$ NMR (100 MHz, $CDCl_3$, $\delta$): 13.42; 14.48; 42.81; 43.29; 104.54; 113.86; 129.50; 137.85; 147.65; 151.00; 152.87. HRMS (ESI): m/z: calcd for $C_{11}H_{13}IN_3O_2$ $[M+H]^+$: 346.00524, found 346.00478.

**2-Cyano-4-{5-[(1,2,3,4-tetrahydroacridin-9-yl)amino]pent-1-yn-1-yl}pyridin-3-yl N,N-diethylcarbamate (20)**

Compound **20** was obtained accordingly to the ***general procedure 1*** using **18** (0.22 g, 0.000637 mol, 1 equiv), **19** (0.202 g, 0.000701 mol, 1.1 equiv), $Pd(PPh_3)_4$ (0.057 g, 0.0000637 mol, 0.1 equiv), CuI (0.024 g, 0.0001275 mol, 0.2 equiv), 5 mL of $Et_3N$ and 8 mL of THF. Compound **20** was purified by column chromatography (silica gel, gradient from ethyl acetate/c-hexane/$Et_3N$ = 20/10/1 to ethyl acetate/$Et_3N$ = 20/1). $R_f$ = 0.14 ($CHCl_3$/MeOH/$Et_3N$ = 100/5/1). Yield 0.267 g, 87%. $^1H$ NMR (400 MHz, $CDCl_3$, $\delta$): 1.14 (3H, t, J =7.2 Hz, $CH_3CH_2$), 1.24 (3H, t, J = 7.2 Hz, $CH_3CH_2$), 1.87-1.94 (6H, m), 2.54 (2H, t, J = 7 Hz, $CH_2$), 2.69 (2H, t, J = 5.6 Hz, $CH_2$), 3.04 (2H, t, J = 5.8 Hz, $CH_2$), 3.30 (2H, q, J = 7.2 Hz, $NCH_2CH_3$), 3.39 (2H, q, J = 7.2 Hz, $NCH_2CH_3$), 3.57 (2H, t, J = 7 Hz, $CH_2NH$), 7.31 (1H, m, ArH), 7.38 (1H, d, J = 4.9 Hz, ArH), 7.53 (1H, m, ArH), 7.89 (2H, d, J = 8.8 Hz, ArH), 8.40 (1H, d, J = 4.9 Hz, ArH). $^{13}C$ NMR (100 MHz, $CDCl_3$, $\delta$): 13.47; 14.26; 17.59; 22.83; 23.15; 25.11; 29.90; 33.86; 42.63; 43.10; 48.07; 74.10; 82.93; 101.32; 114.31; 116.79; 120.42; 122.59; 124.30; 128.67; 128.84; 129.42; 130.09; 147.06; 147.55; 150.52; 151.61; 151.74; 158.52.

**2-Cyano-4-{5-[(1,2,3,4-tetrahydroacridin-9-yl)amino]pentyl}pyridin-3-yl N,N-diethylcarbamate (21)**

To the degazed solution of **20** (0.1 g, 0.208 mmol, 1 equiv) in 2 mL of ethyl acetate Pd/C (10%, 0.033 g, 0.031 mmol, 0.15 equiv) was added, after the degazation the solution was filled with $H_2$. The reaction mixture was stirred at room temperature under $H_2$ (1 atm) for 20h. The solution was filtered through Celite®, the solvent was removed in *vacuum,* and product was dried in *vacuum* overnight. Yield 0.1 g, 99 %. $^1H$ NMR (300 MHz, $CDCl_3$, $\delta$): 1.19 (3H, t, J =7.2 Hz, $CH_3CH_2$), 1.28-1.39 (3H + 2H, t + m, J = 7.2 Hz), 1.64-1.81 (8H, m), 2.40 (2H, m, $CH_2$), 2.66 (2H, t, J = 7.2 Hz, $CH_2$), 3.00 (2H, m, $CH_2$), 3.37 (2H, q, J = 7.2 Hz, $NCH_2CH_3$), 3.50 (2H, q, J = 7.2 Hz, $NCH_2CH_3$), 3.83 (2H, t, J = 6.3 Hz, $CH_2NH$), 7.35-7.39 (2H, m, ArH), 7.61 (1H, t, J = 7.2 Hz, ArH), 7.92 (1H, d, J = 8.4 Hz, ArH), 8.07 (1H, d, J = 9 Hz, ArH), 8.37 (1H, d, J = 5.1 Hz, ArH). $^{13}C$ NMR (100 MHz, $CDCl_3$, $\delta$): 13.44, 14.36, 20.79, 21.96, 23.64, 25. 61, 27.58, 28.27, 29.17, 30.29, 42.75, 43.19, 47.98, 111.42, 114.74, 115.95, 120.52, 124.48, 125.33, 128.53, 129.67, 132.65,

138.88, 146.11, 147.75, 150.45, 151.35, 152.52, 155.99. MS m/z (ESI) calcd. for $C_{29}H_{36}N_5O_2$ $[M+H]^+$, 486.28; found, 486.2.

**N',3-dihydroxy-4-{5-[(1,2,3,4-tetrahydroacridin-9-yl)amino]pentyl}pyridine-2-carboximidamide (6)**

Compound 6 was synthesized accordingly to the ***general procedure* 2** using **21** (0.03 g, 0.06177 mmol, 1 equiv), $NH_2OH.HCl$ (0.129 g, 1.85 mmol, 30 equiv.), pyridine (0.147 g, 0.45 mL, 5.56 mmol, 30 equiv) and 2 mL of ethanol. The product was purified by preparative HPLC chromatography ($CH_3CN$, $H_2O$, TFA 0.1%, gradient $H_2O$ from 95% to 5%). Yield 5 mg, 20%. $^1H$ NMR (300 MHz, MeOD-$D_4$, δ): 1.37-1.47 (2H, m), 1.64-1.73 (2H, qui, J = 7.3 Hz, $CH_2CH_2$), 1.80-1.95 (2H+4H, qui + m, J = 7.3 Hz, $CH_2CH_2$), 2.60 (2H, m, $CH_2$), 2.67 (2H, t, J = 7.3 Hz, $CH_2$), 2.99 (2H, m, $CH_2$), 3.94 (2H, t, J = 7 Hz, $CH_2$), 7.05 (1H, d, J = 4.7 Hz, ArH), 7.53-7.59 (1H, td, J = 7 Hz, J = 1.4 Hz, ArH), 7.73-7.75 (1H, m, ArH), 7.82-7.86 (1H, m, ArH), 7.90 (1H, d, J = 4.7 Hz, ArH), 8.34 (1H, d, J = 8.6 Hz, ArH). $^{13}C$ NMR (100 MHz MeOD-$D_4$, δ): 21.98, 23.08, 24.94, 26.91, 29.12, 29.41, 29.82, 31.17, 112.97, 117.20, 120.29, 126.45, 126.58, 126.75, 133.02, 134.22, 139.81, 139.89, 140.21, 151.82, 154.82, 154.15, 155.31, 158.19. MS m/z (ESI) calcd. for $C_{24}H_{30}N_5O_2$ $[M+H]^+$, 420.23 ; found, 420.2.

**3-(Benzyloxy)-6-{5-[(1,2,3,4-tetrahydroacridin-9-yl)amino]pent-1-yn-1-yl}pyridine-2-carbonitrile (23)**

Compound 23 was obtained accordingly to the ***general procedure 1*** for Sonogashira coupling reaction using 22 (0.099 g, 0.000345 mol, 1 equiv), 19 (0.100 g, 0.0003797 mol, 1.1 equiv), $Pd(PPh_3)_4$ (0.031g, 0.0000345 mol, 0.1 equiv), CuI (0.013 g, 0.000069 mol, 0.2 equiv), $Et_3N$ (3 mL), and THF (3 mL). The crude product was purified by the column chromatography (silica gel, gradient from ethyl acetate to ethyl acetate/MeOH = 8/2, $R_f$ = 0.28). Yield 0.140 g, 86 %. $^1H$ NMR (400 MHz, $CD_3OD$, δ): 1.89-1.92 (4H, m, $CH_2$); 2.09 (2H, qui, J = 6.7 Hz, $CH_2$); 2.59 (2H, t, J = 6.7 Hz, $CH_2$); 2.75 (2H, t, J = 5.5 Hz, $CH_2$); 2.97 (2H, t, J = 5.8 Hz, $CH_2$); 4.08 (2H, t, J = 6.7 Hz, $CH_2$); 5.33 (2H, s, $OCH_2$); 7.34-7.52 (7H, m, ArH); 7.66 (1H, d, J = 8.9 Hz, ArH); 7.70-7.72 (2H, m, ArH); 8.36 (1H, d, J = 8.7 Hz, ArH). $^{13}C$ NMR (100 MHz $CD_3OD$, δ): 17.30; 22.16; 23.23; 25.39; 29.79; 30.28; 30.43; 30.78; 48.16; 72.44; 90.78; 117.96; 119.83; 121.60; 123.59; 125.98; 126.21; 128.71; 129.68; 129.84; 132.84; 133.21; 151.89.

**3-Hydroxy-6-{5-[(1,2,3,4-tetrahydroacridin-9-yl)amino]pentyl}pyridine-2-carbonitrile (24)**

Compound **24** was obtained accordingly to the ***general procedure* 3** for the one-pot reduction of the triple bond and O-debenzylation using **23** (0.10 g, 0.0002116 mol, 1equiv), $Pd(OH)_2/C$ (20%, 0.089 g, 0.0001269 mol, 0.6 equiv), and 2 mL of MeOH. Yield 0.080 g, 80%. $R_f$ = 0.34 ($CH_2Cl_2$/MeOH =10/1). $^1H$ NMR (300 MHz, $CD_3OD$, δ): 1.35-1.45 (2H, m, $CH_2$); 1.65-1.75 (2H, m, $CH_2$); 1.79-1.89 (2H, m, $CH_2$); 1.94 (4H, m, $CH_2$); 2.63-2.68 (4H, m, $CH_2$); 3.02 (2H, m, $CH_2$); 3.93 (2H, t, J = 7 Hz, $CH_2$); 7.19-7.27 (2H, dd, J = 8.7 Hz, ArH); 7.53-7.58 (1H, m, ArH); 7.76-7.85 (2H, m, ArH); 8.34 (1H, d, J = 8.7 Hz, ArH).

**N',3-dihydroxy-6-{5-[(1,2,3,4-tetrahydroacridin-9-yl)amino]pentyl}pyridine-2-carboximidamide (7)**

$NH_2OH.HCl$ (0.015 g, 0.0002098 mol, 5 equiv) and $Na_2CO_3$ (0.011 g, 0.0001049 mol) were stirred in 0.2 mL of water. Then this solution was added to nitrile **24** (0.02 g, 0.00004196 mol, 1 equiv) and the reaction mixture was stirred overnight. The solvents were evaporated, and the crude product was dried in *vacuum.* The purification by the column chromatography (silica gel, $CH_2Cl_2$/MeOH = 10/1) resulted in pure amidoxime 7 with 65% yield. $R_f$ = 0.42 ($CH_2Cl_2$/MeOH = 9/1). $^1H$ NMR (500 MHz, $CD_3OD$, δ): 1.36-1.42 (2H, m, $CH_2$), 1.74-1.84 (2H+2H, m+m, $CH_2$), 1.89-1.94 (4H, m, $CH_2$), 2.57 (2H, m, $CH_2$), 2.70 (2H, t, J = 7.1 Hz, $CH_2$), 2.99 (2H, m, $CH_2$), 3.89 (2H, t, J = 6.9 Hz, $CH_2$), 6.99 (1H, d, J = 8.4 Hz, ArH), 7.03 (1H, d, J = 8.4 Hz, ArH); 7.51-7.54 (1H, m, ArH); 7.74-7.76 (1H, m, ArH); 7.79-7.82 (1H, m, ArH); 8.30 (1H, d, J = 8.7 Hz, ArH). $^{13}C$ NMR (125 MHz, $CD_3OD$, δ): 22.11; 23.13; 25.02; 26.73; 29.77; 29.86; 31.23; 37.51; 113.26; 117.51; 120.88; 125.58; 125.64; 126.30; 126.44; 132.53; 133.86; 140.49; 152.34; 152.49; 153.60; 155.10; 157.75. HRMS m/z (ESI) calcd. for $C_{24}H_{30}N_5O_2$ $[M+H]^+$, 420.2399; found, 420.2433.

**2-Cyano-4-formylpyridin-3-yl N,N-diethylcarbamate** (25)

To the solution of 17 (0.5 g, 2.28 mmol, 1 equiv) in 20 mL of THF at -78 °C the solution of *n*-BuLi (2.5M, in hexanes, 1.37 mL, 3.42 mmol, 1.5 equv) was added dropwise. After 30 min of stirring at -78 °C solution of DMF (1.17 g, 15.9 mmol, 7 equiv) in 5 mL of THF was added. The reaction mixture was stirred at -78 °C for 2.5 h. The reaction was quenched with 10 mL of saturated solution of $NH_4Cl$, the organic phase was separated, and the product was extracted from the aqueous phase with ethyl acetate. The organic phases were combined and dried over $Na_2SO_4$. After removal of the solvent under reduced pressure the crude was subjected to the column chromatography on $Al_2O_3$ basic ($CHCl_3$/Me-

OH/$Et_3N$ = 100/0.5/0.5, $R_f$ = 0.32). Yield 0.3g, 53%. $^1$H NMR (500 MHz, $CDCl_3$, δ): 1.23 (3H, t, J = 7.1 Hz, $CH_3CH_2N$), 1.35 (3H, t, J = 7.1 Hz, $CH_3CH_2N$), 3.40 (2H, q, J = 7.1 Hz, $NCH_2CH_3$), 3.56 (2H, q, J = 7.1 Hz, $NCH_2CH_3$), 7.89 (1H, d, J = 4.7 Hz, ArH), 8.72 (1H, d, J = 4.7 Hz, ArH), 10.08 (1H, s, CHO). $^{13}$C NMR (125 MHz, $CDCl_3$, δ): 13.19; 14.20; 42.74; 43.28; 113.89; 125.42; 130.79; 135.78; 148.65; 150.33; 151.83; 185.78.

**2-Cyano-4-{[methyl({2-[(1,2,3,4-tetrahydroacridin-9-yl)amino]ethyl}) amino]methyl}pyridin-3-yl N,N-diethylcarbamate (28)**

Amine **26** (0.2 g, 0.78 mmol, 1.05 equiv) and aldehyde **25** (0.184 g, 0.744 mmol, 1 equiv) were stirred in 3 mL of THF for 10 min, then $NaBH(OAc)_3$ (0.232 g, 1.09 mmol, 1.4 equiv) was added. The reaction mixture was stirred at room temperature for 2.5 h, then saturated solution of $NaHCO_3$ (0.2 mL) was added. The solvent was evaporated and the residue was purified by the column chromatography (silica gel, $CHCl_3$/MeOH/$Et_3N$ = 100/0.5/0.5, $R_f$ = 0.37). Yield of **28** 0.272 g, 75%. $^1$H NMR (400 MHz, $CDCl_3$, δ): 1.18 (3H, t, J = 7 Hz, $CH_3CH_2N$), 1.29 (3H, t, J = 7 Hz, $CH_3CH_2N$), 1.83-1.85 (4H, m, $CH_2CH_2CH_2CH_2$), 2.24 (3H, s, $CH_3N$), 2.62 (2H, t, J = 5.8 Hz, $CH_2CH_2$), 2.66 (2H, t, J = 5.6 Hz, $CH_2CH_2$), 3.01 (2H, t, J = 5.6 Hz, $CH_2CH_2$); 3.34 (2H, q, J = 7.2 Hz, $NCH_2CH_3$), 3.47 (2H, q, J = 7.2 Hz, $NCH_2CH_3$), 3.55-3.59 (2H + 2H, s + m, $ArCH_2NMe$ + $CH_2N$), 4.93 (1H, br. s., NH); 7.26-7.29 (1H, m, ArH); 7.48-7.52 (1H, m, ArH); 7.63 (1H, d, J = 4.8 Hz, ArH); 7.88-7.91 (1H, m, ArH); 8.45 (1H, d, J = 4.8 Hz, ArH). $^{13}$C NMR (100 MHz, $CDCl_3$, δ): 13.33; 14.35; 22.70; 23.05; 24.93; 33.55; 42.13; 42.58; 43.05; 46.06; 55.31; 58.04; 114.62; 115.65; 119.96; 122.83; 123.90; 127.61; 128.16; 128.78; 129.62; 143.57; 146.73; 147.86; 150.16; 151.19; 151.91; 157.99. HRMS m/z (ESI) calcd. for $C_{28}H_{35}N_6O_2$ $[M+H]^+$, 487.28214; found, 487.29518.

**2-cyano-4-{[methyl({3-[(1,2,3,4-tetrahydroacridin-9-yl)amino]propyl})amino] methyl}pyridin-3-yl N,N-diethylcarbamate (29)**

Amine 27 (0.060 g, 0.000222 mol, 1.1 equiv) and aldehyde **25** (0.05 g, 0.000202 mol, 1 equiv) were stirred in 1 mL of THF for 10 min, then $NaBH(OAc)_3$ (0.060 g, 0.0002827 mmol, 1.4 equiv) was added. The reaction mixture was stirred at room temperature for 2.5 h, then saturated solution of $NaHCO_3$ (0.1 mL) was added. The solvent was evaporated and the residue was purified by the column chromatography (silica gel, $CHCl_3$/MeOH/$Et_3N$ = 100/01/0.5, $R_f$ = 0.57). Yield of 29 0.100 g, 99%. $^1$H NMR (400 MHz, $CDCl_3$, δ): 1.20 (3H, t, J=7.2 Hz, $CH_3CH_2$), 1.30 (3H, t, J=7.2 Hz); 1.80-1.89 (2H + 4H, qui + m, J=6.7 Hz, $CH_2$), 2.19 (3H, s, $CH_3N$), 2.46 (2H, t, J=6.7 Hz), 2.59 (2H, t, J=5.9 Hz), 3.03 (2H, t, J=5.9 Hz), 3.34-3.39 (2H, q, J=7.2 Hz), 3.45-3.50 (2H+2H, s+quart, J=7.2), 3.55 (2H, t, J=6.5 Hz), 7.26-7.30 (1H, m, J=6.8 Hz, J=1.3 Hz), 7.50-7.54 (1H, m, J=6.8 Hz, J=1.3 Hz), 7.56 (1H, d, J=4.9 Hz), 7.87-7.91 (2H, m), 8.38 (1H, d, J=4.9Hz). $^{13}$C NMR (100 MHz, $CDCl_3$, δ): 13.39; 14.36; 22.81; 23.13; 25.02; 29.04; 33.85; 42.59; 42.75; 43.05; 47.36; 55.42; 55.72; 114.71; 115.83; 120.10; 122.84; 123.84; 127.84; 128.52; 128.63; 129.54; 144.01; 147.13; 147.84; 150.16; 150.92; 152.03; 158.24.

**(Z)-N',3-dihydroxy-4-{[methyl({2-[(1,2,3,4-tetrahydroacridin-9-yl)amino]ethyl}) amino] methyl}pyridine-2-carboximidamide (8)**

Compound 8 was synthesized accordingly to the *general procedure* 2 using **28** (0.03 g, 0.0000616 mol), $NH_2OH.HCl$ (0.128 g, 0.001849 mol, 30 equiv), pyridine (0.149 mL, 0.001849 mol, 30 equiv). The crude product was purified by preparative HPLC ($CH_3CN$, $H_2O$, 0.01 M $HCOONH_4$, $NH_4OH$, pH 9, gradient $H_2O$ from 95% to 5%). Yield 8 mg, 32%. $^1$H NMR (400 MHz, $CD_3OD$, δ): 1.93-1.96 (4H, m), 2.63 (2H, t, J=5.5. Hz), 3.01 (3H, s, $CH_3N$), 3.03 (2H, m), 3.55 (2H, t, J=5.1 Hz), 4.31-4.33 (2H+2H, t+s, $CH_2$), 7.23 (1H, d, J =4.6 Hz), 7.54-7.57 (1H, m, ArH), 7.76-7.78 (2H, m), 7.83-7.87 (1H, m), 8.21 (1H, d, J=8.8 Hz). $^{13}$C NMR (100 MHz, $CD_3OD$, δ): 17.42; 18.85; 21.84; 22.89; 24.86; 29.38; 43.61; 55.97; 56.21; 57.00; 117.05; 120.51; 126.01; 126.72; 127.76; 133.62; 134.21; 139.72; 140.09; 154.94; 157.11. HRMS (ESI, m/z): calcd. for $C_{23}H_{29}N_6O_2$ $[M+H]^+$ 421.23519; found, 421.23561.

**(Z)-N',3-dihydroxy-4-{[methyl({3-[(1,2,3,4-tetrahydroacridin-9-yl)amino]propyl}) amino] methyl}pyridine-2-carboximidamide (9)**

Compound 9 was obtained accordingly to the ***general procedure* 2** using **29** (0.050 g, 0.00009986 mol, 1 equiv), $NH_2OH.HCl$ (0.208 g, 0.002995 mol, 30 equiv), pyridine (0.24 mL, 0.002996 mol, 30 equiv). The crude product was purified by preparative HPLC ($CH_3CN$, $H_2O$, 0.01 M $HCOONH_4$, $NH_4OH$, pH 9, gradient $H_2O$ from 95% to 5%). Yield 17 mg, 37%. $^1$H NMR (400 MHz, $CD_3OD$, δ): 1.93-1.96 (4H, m); 2.28-2.36 (2H, m, $CH_2$); 2.60 (2H, m, $CH_2$); 2.93 (3H, s, $CH_3N$); 3.04 (2H, m, $CH_2$); 3.25-3.29 (2H, m, $CH_2$); 4.04 (2H, t, J = 6.7 Hz, $CH_2$); 4.41 (2H, s, $CH_2$); 7.32 (1H, d, J = 4.8 Hz, ArH), 7.56-7.60 (1H, m, ArH), 7.78-7.80 (1H, dd, J = 8.5 Hz, J = 1 Hz, ArH); 7.84-7.89 (1H, m, ArH); 8.03 (1H, d, J = 4.8 Hz, ArH); 8.31 (1H, d, J = 8.5 Hz, ArH). $^{13}$C NMR (100 MHz, $CD_3OD$, δ): 21.86; 23.03; 25.20; 26.27;

29.48; 41.91; 45.68; 54.23; 55.19; 113.52; 117.32; 120.59; 126.11; 126.55; 126.91; 128.08; 134.31; 139.79; 140.45; 152.51; 155.07; 155.33; 157.80. HRMS (ESI, m/z): calcd. for $C_{24}H_{31}N_6O_2$ $[M+H]^+$ 435.25084; found, 435.25099.

**Methyl 3-hydroxy-4-{5-[(1,2,3,4-tetrahydroacridin-9-yl)amino]pentyl}pyridine-2-carboxylate (30)**

To the solution of 21 (0.140 g, 0.000288 mol) in dry MeOH (2 mL) 0.3 mL of $H_2SO_4$ (98%) was added dropwise. The reaction mixture was refluxed during 24 h. After cooling to the room temperature the reaction mixture was quenched with concentrated solution of $Na_2CO_3$. The product was extracted with $CH_2Cl_2$ and purified by the column chromatography ($CH_2Cl_2$/MeOH = 10/1) to give **30** with 42% yield. $R_f$ = 0.45 ($CH_2Cl_2$/MeOH = 10/1). $^1$H NMR (500 MHz, $CDCl_3$, $\delta$): 1.39-1.46 (2H, m, $CH_2$); 1.64 (2H, qui, J = 7.7 Hz, $CH_2$); 1.74-1.83 (6H, m, $CH_2$); 2.61 (2H, t, J = 6.1 Hz, $CH_2$); 2.64 (2H, t, J = 7.7 Hz, $CH_2$); 3.05 (2H, t, J = 5.7 Hz); 3.69 (2H, t, J = 7.1 Hz); 3.98 (3H, s, $CH_3O$); 5.49 (1H, br.s., NH); 7.19 (1H, d, J = 4.4 Hz, ArH); 7.29-7.33 (1H, m, ArH); 7.51-7.54 (1H, m, ArH); 8.03-8.05 (2H, m, ArH); 8.09 (1H, d, J = 4.4 Hz, ArH). $^{13}$C NMR (125 MHz, $CDCl_3$, $\delta$): 21.68; 22.52; 24.38; 26.55; 28.23; 29.01; 30.88; 31.08; 48.72; 53.29; 113.25; 117.78; 123.88; 124.08; 124.53; 129.14; 129.48; 130.68; 140.60; 141.22; 142.51; 153.69; 154.24; 157.64; 170.44.

**3-Hydroxy-4-{5-[(1,2,3,4-tetrahydroacridin-9-yl)amino]pentyl]pyridine-2-carbaldehyde (31)**

Aldehyde **31** was obtained from methyl ester **30** accordingly to the ***general procedure 4*** and purified by the column chromatography (silica gel, ethyl acetate/MeOH = 10/1) with yield 33% (over three steps). $R_f$ = 0.35 (ethyl acetate/MeOH = 11/3). $^1$H NMR (300 MHz, $CDCl_3$, $\delta$): 1.40-1.50 (2H, m, $CH_2$); 1.62-1.77 (4H, m, $CH_2$); 1.84-1.91 (4H, m, $CH_2$); 2.64-2.71 (4H, m, $CH_2$); 3.07 (2H, t, $CH_2$); 3.54 (2H, t, J = 7.2 Hz, $CH_2$); 7.20-7.23 (1H, m, ArH); 7.30-7.35 (1H, td, J = 7.3 Hz, J = 0.9 Hz, ArH); 7.52-7.57 (1H, td, J = 6.9 Hz, J = 0.9 Hz); 7.93-7.98 (2H, m, ArH); 8.21 (1H, d, J = 4.5 Hz, ArH); 10.04 (1H, s,CHO).

**2-[(hydroxyimino)methyl]-4-{5-[(1,2,3,4-tetrahydroacridin-9-yl)amino]pentyl} pyridin-3-ol (10)**

Compound 10 was obtained accordingly to the ***general procedure 5*** using **31** (0.015 g, 0.03851 mmol, 1 equiv), $NH_2OH.HCl$ (0.004g, 0.05777 mmol, 1.5 equiv), $CH_3COONa$ (0.005 g, 0.05777 mmol, 1.5 equiv), and 1 mL of ethanol. The crude product was purified by the column chromatography (silica gel, ethyl acetate/MeOH = 10/1) to give **10** with 51% yield. $R_f$ = 0.44 (ethyl acetate/MeOH = 2/1). $^1$H NMR (300 MHz, $CD_3OD$, $\delta$): 1.37-1.47 (2H, m, $CH_2$); 1.62-1.80 (2H+2H, m+m, $CH_2$); 1.90-1.93 (4H, m, $CH_2$); 2.66-2.71 (4H, m, $CH_2$); 2.99 (2H, m, $CH_2$); 3.71 (2H, t, J = 6.9 Hz, $CH_2$); 7.08 (1H, d, J = 4.8 Hz, ArH); 7.40-7.46 (1H, m, ArH); 7.64-7.69 (1H, m, ArH); 7.74-7.77 (1H, m, ArH); 7.96 (1H, d, J = 4.8 Hz, ArH); 8.18 (1H, d, J = 8.7 Hz, ArH); 8.26 (1H, s, CH=N). $^{13}$C NMR (125 MHz, $CD_3OD$ $\delta$): 23.17; 23.83; 25.82; 27.49; 29.39; 29.96; 30.92; 31.81; 32.53; 115.45; 119.88; 125.29; 125.43; 126.48; 131.48; 136.41; 140.74; 141.65; 153.01; 154.31; 155.14; 156.52. HRMS (ESI, m/z): calcd. for $C_{24}H_{29}N_4O_2$ $[M+H]^+$ 405.2290; found, 405.2301.

**Methyl 3-(benzyloxy)-6-{5-[(1,2,3,4-tetrahydroacridin-9-yl)amino]pent-1-yn-1-yl}-pyridine-2-carboxylate (35)**

Compound 35 was obtained accordingly to the ***general procedure 1*** for Sonogashira coupling reaction using 32 (0.147 g, 0.4556 mmol, 1 equiv), 19 (0.132 g, 0.502 mmol, 1.1 equiv), $Pd(PPh_3)_4$ (0.041g, 0.04556 mmol, 0.1 equiv), CuI (0.017 g, 0.09112 mmol, 0.2 equiv), $Et_3N$ (3 mL), and THF (6 mL). The crude product was purified by the column chromatography (silica gel, gradient from ethyl acetate to ethyl acetate / MeOH = 8/2, $R_f$ = 0.24). Yield 180 mg, 78%. $^1$H NMR (300 MHz, $CDCl_3$, $\delta$): 1.76-1.84 (4H, m), 1.87-1.97 (2H, qui, J=6.7 Hz), 2.49 (2H, t, J=6.7 Hz), 2.64 (2H, m), 3.03 (2H, m), 3.67 (2H, t, J=6.9 Hz), 3.88 (3H, s, $CH_3O$), 5.14 (2H, s, $PhCH_2O$), 7.18-7.38 (8H, m, ArH), 7.48 (1H, t, J=7.6 Hz), 7.90-7.93 (2H, m). $^{13}$C NMR (100 MHz, $CDCl_3$, $\delta$): 17.29; 22.79; 23.13; 25.05; 29.98; 33.79; 48.42; 52.88; 71.10; 80.64; 88.84; 120.24; 121.96; 122.93; 124.19; 127.12; 128.37; 128.47; 128.63; 128.75; 128.83; 128.95; 130.11; 132.24; 132.34; 135.23; 135.66; 146.91; 150.97; 153.20; 158.23; 164.99.

**Methyl 3-hydroxy-6-{5-[(1,2,3,4-tetrahydroacridin-9-yl)amino]pentyl}pyridine-2-carboxylate (38)**

Compound 38 was obtained accordingly to the ***general procedure*** **3** for the reduction of the triple bond and O-debenzylation using 35 (0.180 g, 0.355 mmol), $Pd(OH)_2$/C (20%, 0.075 g, 0.1066 mmol, 0.3 equiv), and 2.5 mL of MeOH. Yield 0.140 g, 94%. $R_f$ = 0.34 ($CH_2Cl_2$/MeOH =10/1). $^1$H NMR (300 MHz, $CDCl_3$, $\delta$): 1.40-1.50 (2H, m), 1.67-1.78 (4H, m), 1.87-1.92 (4H, m), 2.63 (2H, m), 2.78 (2H, t, J=7.9 Hz), 3.11 (2H, m), 3.56 (2H, t, J=7.2 Hz), 4.00 (3H, s, $CH_3O$), 7.23 (1H, d, J = 8.6 Hz), 7.28 (1H, d, J=8.6 Hz), 7.31-7.36 (1H, m), 7.54-7.59 (1H, m), 7.95 (1H, d, J=8 Hz), 8.05 (1H, d, J=8.6 Hz). $^{13}$C NMR (100 MHz, $CDCl_3$, $\delta$): 20.87; 22.11; 23.78; 26.38; 28.60; 29.62; 31.09; 37.02; 48.61; 53.55; 110.88; 115.99; 121.66; 124.19; 125.49; 127.88; 129.65; 132.63; 139.27; 152.03; 153.45; 155.55. HRMS (ESI, m/z): calcd. for $C_{25}H_{30}N_3O_3$ $[M+H]^+$, 420.22871; found, 420.22836.

**3-Hydroxy-6-{5-[(1,2,3,4-tetrahydroacridin-9-yl)amino]pentyl}pyridine-2-carbaldehyde (41)**

Compound **41** was obtained by the ***general procedure 4*** from **38** with yield 37% (over three steps). $R_f$ = 0.23 (ethyl acetate/MeOH = 10/3). $^1H$ NMR (400 MHz, $CDCl_3$, δ): 1.40-1.48 (2H, m), 1.67-1.79 (4H, m), 1.85-1.90 (4H, m), 2.64 (2H, m), 2.76 (2H, t, J=7.7 Hz), 3.05 (2H, m), 3.52 (2H, t, J=7.3 Hz), 7.23 (1H, d, J=8.7 Hz), 7.25 (1H, d, J=8.7 Hz), 7.29-7.33 (1H, m, ArH), 7.51-7.55 (1H, m, ArH), 7.92-7.95 (2H, m), 9.99 (1H, s, CHO). $^{13}C$ NMR (100 MHz, $CDCl_3$, δ): 22.73; 23.11; 24.88; 26.70; 29.48; 31.76; 33.48; 37.28; 49.45; 115.48; 123.10; 124.02; 126.64; 127.99; 129.01; 129.88; 131.06; 135.95; 151.45; 154.66; 157.23; 157.88; 198.83. HRMS (ESI, m/z): calcd. for $[M+H]^+$, $C_{24}H_{28}N_3O_2$, 390.21815; found, 390.21892.

**2-[(1E)-(hydroxyimino)methyl]-6-{5-[(1,2,3,4-tetrahydroacridin-9-yl)amino]pentyl} pyridin-3-ol (11)**

Compound **11** was obtained accordingly to the ***general procedure 5*** using **41** (0.050g, 0.00012837 mol, 1 equiv), $NH_2OH.HCl$ (0.013g, 0.00019256 mol, 1.5 equiv), $CH_3COONa$ (0.016 g, 0.00019256 mol, 1.5 equiv), and 1.2 mL of ethanol. After evaporation of the solvent and purification by column chromatography (ethyl acetate/MeOH = 8/2) product was obtained with yield 55%. $R_f$ = 0.44 (ethyl acetate/MeOH = 2/1). $^1H$ NMR (400 MHz, $CD_3OD$, δ): 1.37-1.44 (2H, m, $CH_2$), 1.68-1.76 (2H, qui, J = 7.4 Hz, $CH_2$), 1.77-1.85 (2H, qui, J = 7.4 Hz, $CH_2$), 1.90-1.94 (4H, qui, J = 3.3 Hz, $CH_2$), 2.63 (2H, m, $CH_2$), 2.70 (2H, t, J = 7.4 Hz, $CH_2$), 3.00 (2H, m, $CH_2$), 3.87 (2H, t, J = 6.9 Hz, $CH_2$), 7.08 (1H, d, J = 8.4 Hz, ArH), 7.20 (1H, d, J = 8.4 Hz, ArH), 7.50-7.54 (1H, m, ArH), 7.76-7.81 (2H, m, ArH), 8.17 (1H, s, N=CH), 8.30 (1H, d, J = 8.7 Hz). $^{13}C$ NMR (100 MHz, $CD_3OD$, δ): 22.31; 23.30; 25.25; 27.05; 30.30; 30.53; 30.91; 31.42; 37.56; 113.66; 117.95; 118.57; 121.70; 125.51; 126.08; 126.18; 133.42; 136.35; 152.90; 153.85; 154.49. MS m/z (ESI) calcd. for $C_{24}H_{29}N_4O_2$ $[M+H]^+$, 405.22 ; found, 405.2.

**Methyl 3-(benzyloxy)-6-{4-[(1,2,3,4-tetrahydroacridin-9-yl)amino]but-1-yn-1-yl}pyridine-2-carboxylate (36)**

Compound 36 was obtained accordingly to the ***general procedure 1*** for Sonogashira coupling reaction using 32 (0.292g, 0.0009078 mol, 1 equiv), **34** (0.250g, 0.0009986 mol, 1.1 equiv), $Pd(PPh_3)_4$ (0.081g, 0.00009078 mol, 0.1 equiv), CuI (0.035 g, 0.00018156 mol, 0.2 equiv), $Et_3N$ (6 mL), and THF (12 mL). The crude product was purified by the column chromatography (silica gel, gradient from ethyl acetate/MeOH = 10/1 to ethyl acetate / MeOH = 8/2), $R_f$ = 0.43 (ethyl acetate / MeOH = 4/1). Yield 0.440 g, 98%. $^1H$ NMR (400 MHz, $CDCl_3$, δ): 1.82-1.89 (4H, m), 2.67 (2H, t, J= 6.3 Hz), 2.79 (2H, t, J= 5.9 Hz), 3.06 (2H, t, J= 5.9 Hz), 3.70-3.75 (2H, dt, J= 6.4 Hz), 3.95 (3H, s), 4.48 (1H, t, J= 6.4 Hz, NH), 5.19 (2H, s, $OCH_2Ph$), 7.25 (1H, d, J= 8.8 Hz), 7.28 (2H, d, J= 8.8 Hz), 7.31-7.42 (6H, m, ArH), 7.52-7.56 (1H, m), 7.97 (2H, t, ArH, J= 8.3 Hz).$^{13}$ C NMR (100 MHz, $CDCl_3$, δ): 22.10; 22.59; 22.99; 24.93; 29.89; 33.29; 47.34; 52.93; 71.10; 81.98; 86.71; 116.94; 120.15; 121.92; 123.00; 124.56; 127.14; 127.66; 128.51; 128.97; 129.30; 130.09; 134.74; 135.59; 140.67; 146.04; 151.08; 153.37; 157.78; 164.98. HRMS (ESI, *m*/*z*): calcd. for $C_{31}H_{30}N_3O_3$ $[M+H]^+$, 492.22871; found, 492.22851.

**Methyl 3-hydroxy-6-{4-[(1,2,3,4-tetrahydroacridin-9-yl)amino]butyl}pyridine-2-carboxylate (39)**

Compound **39** was obtained accordingly to the ***general procedure 3*** using **36** (0.430g, 0.0008747 mol), $Pd(OH)_2/C$ (20%, 0.184 g, 0.0002624 mol, 0.3 equiv), and 3 mL of MeOH. Yield 0.340 g, 96%. $R_f$ = 0.43 ($CH_2Cl_2$/MeOH =10/1). $^1H$ NMR (300 MHz, $CD_3OD$, δ): 1.67-1.74 (4H, m), 1.87-1.91 (4H, m), 2.68 (2H, t, J = 5.3 Hz), 2.72 (2H, t, J= 7 Hz), 2.96 (2H, t, J= 5.5 Hz), 3.62 (2H, t, J= 6.7 Hz), 3.96 (3H, s, $CH_3O$), 7.23 (1H, d, J = 8.6 Hz), 7.27 (2H, d, J=8.6 Hz), 7.34-7.40 (1H, m), 7.57-7.62 (1H, m), 7.74 (1H, d, J= 8.4 Hz), 8.10 (1H, d, J= 8.4 Hz). $^{13}C$ NMR (100 MHz, $CD_3OD$, δ): 23.25; 23.87; 25.96; 28.22; 31.31; 32.84; 37.27; 53.34; 115.73; 120.15; 125.07; 125.29; 125.74; 128.52; 130.46; 130.65; 131.09; 145.48; 154.49; 154.54; 156.94; 158.65; 170.62. HRMS (ESI, *m*/*z*): calcd. for $C_{24}H_{28}N_3O_3$ $[M+H]^+$, 406.21306; found, 406.21307.

**3-Hydroxy-6-{4-[(1,2,3,4-tetrahydroacridin-9-yl)amino]butyl}pyridine-2-carbaldehyde (42)**

Compound **42** was obtained from **39** accordingly to the ***general procedure 4*** after purification by the column chromatography (silica gel, ethyl acetate/MeOH = 8/2) with yield 54% (over 3 steps). $R_f$ = 0.21 (ethyl acetate/MeOH = 7/3). $^1H$ NMR (400 MHz, $CDCl_3$, δ): 1.66-1.75 (2H, m), 1.78-1.89 (6H, m), 2.67 (2H, m), 2.79 (2H, t, J= 7 Hz), 3.04 (2H, m), 3.52 (2H, t, J= 7 Hz), 7.20 (1H, d, J = 8.6 Hz), 7.24 (1H, d, J= 8.6 Hz), 7.31 (1H, td, J= 6.8 Hz, J = 1 Hz), 7.53 (1H, td, J= 6.8 Hz, J= 1 Hz), 7.89-7.93 (2H, m), 9.98 (1H, s, CH=O). $^{13}C$ NMR (100 MHz, $CDCl_3$, δ): 22.89; 23.20; 25.01; 26.96; 31.37; 33.92; 36.98; 49.32; 116.06; 120.29; 122.94; 123.95; 126.69; 128.68; 129.87; 136.00; 147.27; 150.99; 154.29; 157.29; 158.43; 198.74. HRMS (ESI, *m*/*z*): calcd. for $C_{23}H_{26}N_3O_2$ $[M+H]^+$, 376.20250; found, 376.20201.

**2-[1-(hydroxyimino)methyl]-6-{4-[(1,2,3,4-tetrahydroacridin-9-yl)amino]butyl} pyridin-3-ol (12)**

Compound 12 was obtained accordingly to the ***general procedure 5*** using **42** (0.090g, 0.0002397 mol, 1 equiv), $NH_2OH.HCl$ (0.025g, 0.0003596 mol, 1.5 equiv), $CH_3COONa$ (0.029 g, 0.0003596 mol, 1.5 equiv), and 2.5 mL of ethanol. Compound **12** was purified by the column chromatography (silica gel, $CH_2Cl_2$/MeOH = 8/2) with yield 55%. $R_f$= 0.6 (ethyl acetate/MeOH = 2/1). $^1H$ NMR (400 MHz, $CD_3OD$, δ): 1.80-1.83 (4H, m, $CH_2CH_2CH_2CH_2$), 1.90-1.95 (4H, m, $CH_2CH_2CH_2CH_2$), 2.62 (2H, t, J = 5.6 Hz, $CH_2$), 2.71 (2H, t, J = 6.4 Hz, $CH_2$), 2.98 (2H, m, $CH_2$), 3.87 (2H, t, J = 5.6 Hz, $CH_2$), 7.04 (1H, d, J = 8.4 Hz, ArH), 7.09 (1H, d, J = 8.4 Hz); 7.47-7.51 (1H, m, ArH), 7.71-7.79 (2H, m, ArH), 8.08 (1H, s, CH=N), 8.24 (1H, d, J = 8.8 Hz). $^{13}C$ NMR (100 MHz, $CD_3OD$, δ): 22.04; 23.09; 25.03; 27.55; 29.84; 30.60; 37.11; 113.35; 117.54; 121.08; 125.43; 125.83; 126.12; 126.19; 133.51; 136.24; 140.59; 152.44; 152.75; 153.65; 154.12; 157.25. HRMS (ESI, *m/z*): calcd. for $C_{23}H_{27}N_4O_2$ $[M+H]^+$ 391.21340; found, 391.21337.

**Methyl 3-(benzyloxy)-5-{5-[(1,2,3,4-tetrahydroacridin-9-yl)amino]pent-1-yn-1-yl}pyridine-2-carboxylate (37)**

Compound 37 was obtained accordingly to the ***general*** *procedure* ***1*** using **33** (0.333g, 0.001035 mol, 1 equiv), **34** (0.300g, 0.001139 mol, 1.1 equiv), $Pd(PPh_3)_4$ (0.093g, 0.0001035 mol, 0.1 equiv), CuI (0.039 g, 0.0002071 mol, 0.2 equiv), $Et_3N$ (6 mL), and THF (12 mL). The crude product was purified by the column chromatography (silica gel, $CH_2Cl_2$/MeOH = 9/1), $R_f$ = 0.52 ($CH_2Cl_2$/MeOH = 8/2). Yield 87%. $^1H$ NMR (300 MHz, $CDCl_3$, δ): 1.77-1.79 (4H, m, $CH_2$), 2.14-2.24 (2H, m, $CH_2$), 2.63 (2H, t, J = 6.7 Hz), 2.69 (2H, t, J = 5.1 Hz), 3.21 (2H, t, J = 5.1 Hz), 3.94 (3H, s, $OCH_3$), 4.09-4.15 (2H, m), 5.15 (2H, s, $OCH_2$), 6.68 (1H, br.s., NH), 7.28-7.43 (7H, m, ArH), 7.55-7.60 (1H, m, ArH), 8.19 (1H, d, J = 0.9 Hz, ArH), 8.27 (1H, d, J = 8.6 Hz), 8.43 (1H, d, J = 8.6 Hz). $^{13}C$ NMR (100 MHz, $CDCl_3$, δ): 17.32; 20.84; 22.11; 24.86; 28.81; 29.39; 47.19; 52.79; 70.99; 78.30; 94.74; 111.82; 116.19; 120.58; 124.25; 124.70; 125.28; 127.04; 128.32; 128.82; 132.27; 133.87; 134.83; 135.61; 137.82; 138.98; 143.64; 151.33; 154.26; 155.82; 164.83.

**Methyl 3-hydroxy-5-{5-[(1,2,3,4-tetrahydroacridin-9-yl)amino]pentyl}pyridine-2-carboxylate (40)**

Compound **40** was synthesized accordingly to the ***general procedure*** *3* using 37 (0.470g, 0.0009277 mol, 1 equiv), $Pd(OH)_2$/C (20%, 0.195g, 0.0002783 mol, 0.3 equiv), and 4 mL of MeOH. Yield 0.344g, 89%. $R_f$ = 0.29 ($CH_2Cl_2$/MeOH =10/1). $^1H$ NMR (400 MHz, $CDCl_3$, δ): 1.45-1.53 (2H, m, $CH_2$), 1.69-1.76 (2H, m, $CH_2$), 1.81-1.91 (6H, m, $CH_2$), 2.58 (2H, t, J = 6 Hz, $CH_2$), 2.68 (2H, t, J = 7.5 Hz, $CH_2$), 3.31 (2H, t, J = 6 Hz, $CH_2$), 3.90-3.95 (2H, m, $CH_2$), 4.02 (3H, s, $OCH_3$), 5.90 (1H, br.s., NH), 7.15 (1H, d, J = 1.6 Hz, ArH), 7.42 (1H, t, J = 7.7 Hz), 7.66 (1H, t, J = 7.7 Hz), 8.08 (1H, d, J = 1.6 Hz), 8.16 (1H, d, J = 8.5 Hz), 8.57 (1H, d, J = 8.5 Hz), 10.57 (1H, br.s., OH). $^{13}C$ NMR (100 MHz, $CDCl_3$, δ): 20.78; 22.05; 24.42; 26.29; 28.54; 30.11; 30.86; 32.79; 48.17; 53.16; 111.24; 115.93; 120.41; 124.70; 125.27; 128.01; 130.71; 132.41; 138.73; 142.25; 145.07; 151.03; 155.88; 158.77; 169.94.

**3-Hydroxy-5-{5-[(1,2,3,4-tetrahydroacridin-9-yl)amino]pentyl}pyridine-2-carbaldehyde (43)**

Compound **43** was obtained from **40** accordingly to the ***general procedure 4*** after purification by the column chromatography (silica gel, $CH_2Cl_2$/MeOH = 9/1) with yield 44% (over 3 steps). $R_f$ = 0.37 ($CH_2Cl_2$/MeOH = 9/1). $^1H$ NMR (400 MHz, $CDCl_3$, δ): 1.37-1.45 (2H, m, $CH_2$), 1.61-1.70 (4H, m, $CH_2$), 1.84-1.90 (4H, m, $CH_2$), 2.62 (2H, t, J= 7.7 Hz), 2.65 (2H, t, J = 5.6 Hz), 3.03 (2H, t, J = 5.6 Hz), 3.43-3.47 (2H, m, $CH_2$), 7.09 (1H, s, ArH), 7.29-7.33 (1H, m, ArH), 7.49-7.53 (1H, m, ArH), 7.87-7.90 (2H, dd, J = 8.3 Hz, J = 3.6 Hz), 8.12 (1H, s, ArH), 9.99 (1H, s, CHO). $^{13}C$ NMR (100 MHz, $CDCl_3$, δ): 22.89; 23.18; 24.99; 26.65; 30.32; 31.69; 33.24; 33.99; 49.36; 116.15; 120.33; 122.86; 123.93; 125.05; 128.60; 128.71; 135.37; 143.43; 145.95; 147.34; 150.87; 158.46; 158.88; 198.13.

**2-[1-(hydroxyimino)methyl]-5-{5-[(1,2,3,4-tetrahydroacridin-9-yl)amino]pentyl} pyridin-3-ol (13)**

Compound 13 was obtained accordingly to the ***general procedure 5*** using **43** (0.090g, 0.0002397 mol, 1 equiv), $NH_2OH.HCl$ (0.025g, 0.0003596 mol, 1.5 equiv), $CH_3COONa$ (0.029 g, 0.0003596 mol, 1.5 equiv), and 2.5 mL of ethanol. Compound **13** was purified by preparative HPLC ($CH_3CN$, $H_2O$, 0.01M $HCOONH_4$, $NH_4OH$, pH 9), gradient $H_2O$ from 95% to 5%) with yield 54%. $^1H$ NMR (400 MHz, $CD_3OD$, δ): 1.38-1.46 (2H, m, $CH_2$); 1.65-1.72 (2H, m, $CH_2$); 1.80-1.87 (2H, m, $CH_2$); 1.92-1.95 (4H, m, $CH_2$); 2.61-2.65 (4H, m, $CH_2$); 2.99 (2H, t, J = 6 Hz, $CH_2$); 3.92 (2H, t, J = 7 Hz, $CH_2$); 7.14 (1H, d, J = 1.5 Hz, ArH); 7.53-7.57 (1H, td, J = 7 Hz, J = 1.4 Hz, ArH); 7.73-7.76 (1H, m, ArH); 7.79-7.84 (1H, m, ArH); 7.91 (1H, d, J = 1.5 Hz, ArH); 8.24 (1H, s, CH=N); 8.33 (1H, d, J = 8.7 Hz); 8.56 (1H, s, OH). $^{13}C$ NMR (100 MHz, $CD_3OD$, δ): 22.63; 23.49; 25.45; 27.15; 31.20; 31.39; 31.59; 33.35; 49.25; 114.39; 118.73; 123.17; 125.21; 125.77; 125.86; 127.74; 132.58; 135.42; 141.48; 142.20; 142.87; 152.39; 156.30; 170.45. HRMS (ESI, *m/z*): calcd. for $C_{24}H_{29}N_4O_2$ $[M+H]^+$ 405.22905; found, 405.22937.

**N-[5-(pyridin-2-yl)pent-4-yn-1-yl]-1,2,3,4-tetrahydroacridin-9-amine (53)**

To the degazed solution of 2-bromopyridine (0.093 g, 0.000587 mol, 1 equiv) in $CH_3CN/Et_3N$ (9 mL /6 mL) catalysts $Pd(PPh_3)_2Cl_2$ (0.016 g, 0.00002348 mol, 0.04 equiv) and CuI (0.00447 g, 0.00002348 mol, 0.04 equiv) were added. After degazation and bubbling with Ar and $H_2$ the mixture was stirred at the room temperature for 5 min, then the degazed solution of alkyne **19** (0.17 g, 0.000645 mol, 1.1 equiv) was added, and the reaction mixture was stirred at 40 °C during 17 h. After concentration at reduced pressure the residue was purified by the flash chromatography (ethyl acetate / MeOH gradient from 100/0 to 90/10) providing 0.14 g of pure compound **53** with 67% yield. $R_f$= 0.27 (ethyl acetate/MeOH = 9/1). $^1H$ NMR (300 MHz, $CDCl_3$, δ): 1.79 (4H, m, $CH_2$); 2.07 (2H, qui, J = 6.7 Hz, $CH_2$); 2.59 (2H, t, J = 6.7 Hz, $CH_2$); 2.67 (2H, m, $CH_2$); 3.12 (2H, m, $CH_2$); 3.92 (2H, t, J = 6.7 Hz, $CH_2$); 5.52 (1H, br s, NH); 7.17-7.21 (1H, m, ArH); 7.27-7.35 (2H, m, ArH); 7.53-7.63 (2H, m, ArH); 8.12 (1H, d, J = 8.4 Hz, ArH); 8.19 (1H, d, J = 8.4 Hz, ArH); 8.52 (1H, m, ArH). $^{13}C$ NMR (100 MHz, $CDCl_3$, δ): 17.05; 21.45; 22.36; 24.79; 29.39; 30.63; 47.63; 81.71; 89.07; 113.51; 117.65; 122.77; 123.32; 124.05; 124.72; 126.92; 130.74; 136.26; 141.96; 143.29; 149.85; 153.83.

**N-[5-(pyridin-2-yl)pentyl]-1,2,3,4-tetrahydroacridin-9-amine (51)**

To the degazed solution of **53** (0.140g, 0.00041 mol, 1 equiv) in methanol (2 mL) Pearlman's catalyst $Pd(OH)_2/C$ (20%, moisture 50%, 0.086 g, 0.000123 mol, 0.3 equiv) was added. After the degazation the solution was bubbled with $H_2$. The reaction mixture was stirred at RT under $H_2$ at 1 atm for 17 h. The solution was filtered through celite, the solvent was evaporated, and the product was dried in *vacuum.* The crude product was purified by the column chromatography ($CH_2Cl_2$/MeOH = 100/5) to give 0.14 g of **51** with 56% yield. $R_f$ = 0.41 ($CH_2Cl_2$/MeOH = 100/5). $^1H$ NMR (300 MHz, $CDCl_3$, δ): 1.39-1.49 (2H, m, $CH_2$); 1.70-1.92 (8H, m, $CH_2$); 2.61 (2H, J = 5.9 Hz, $CH_2$); 2.75 (2H, J = 7.5 Hz, $CH_2$); 3.21 (2H, J = 5.9 Hz, $CH_2$); 3.88-3.94 (2H, td, J = 6.6 Hz, $CH_2$); 6.49 (1H, t, J = 5.5 Hz, NH); 7.03-7.09 (2H, m, ArH); 7.34-7.39 (1H, m, ArH); 7.51-7.61 (2H, m, ArH); 8.20 (1H, d, J = 8.7 Hz, ArH); 8.41-8.45 (2H, m, ArH). $^{13}C$ NMR (100 MHz, $CDCl_3$, δ): 20.79; 22.04; 24.04; 26.28; 28.52; 29.29; 30.89; 37.92; 48.45; 111. 05; 115.88; 120.57; 121.35; 123.11; 124.65; 125.32; 132.52; 136.74; 138.83; 149.19; 151.16; 155.82; 161.67.

**9-(Pent-4-yn-1-ylsulfanyl)-1,2,3,4-tetrahydroacridine (46)**

To the solution of compound **44** (2.70 g, 12.54 mmol, 1 equiv) and tetra-n-butylammonium bromide (0.50 g, 1.57 mmol, 0.14 equiv) in 70 mL of THF alkylating agent **45** (3.72 mL, 15.78 mmol, 1.25 equiv) was added followed by 50 mL of aqueous solution of NaOH (50%). The reaction mixture was stirred at reflux during 3h. The organic phase was separated, the aqueous phase was extracted with ethyl acetate. The organic phases were combined, washed with water, dried under $Na_2SO_4$ and evaporated. The crude material was purified by flash chromatography to give 1.97 g of **46** (yellow oil, 56% yield). $^1H$ NMR (400 MHz, $CDCl_3$, δ): 8.46 (1H, dd, *J* = 8.4, 1.1 Hz), 7.96 (1H, d, *J*= 8.4 Hz), 7.63-7.59 (1H, m), 7.52-7.48 (1H, m), 3.20 (2H, t, *J*= 6.3 Hz), 3.12 (2H, t, *J* = 6.3 Hz), 2.92 (2H, t, *J* = 7.2 Hz), 2.28 (2H, td, J = 6.9, 2.7 Hz), 1.98-1.86 (5H, m), 1.69 (2H, qui, *J* = 7.1 Hz). $^{13}C$ NMR (100 MHz, $CDCl_3$, δ): 159.27, 146.87, 141.43, 136.08, 129.32, 129.08, 128.87, 126.39, 126.08, 83.19, 69.37, 34.83, 34.48, 29.37, 28.90, 23.28, 22.98, 17.77. HRMS (ESI) m/z: calculated for $C_{18}H_{20}NS$ $[M+H]^+$ 282.13164, found $[M+H]^+$ 282.13062.

**Methyl 3-hydroxy-6-[5-(1,2,3,4-tetrahydroacridin-9-ylsulfanyl)pent-1-yn-1-yl]pyridine-2-carboxylate (48)**

To the degazed solution of **47** (0.75 g, 3.23 mmol, 1 equiv) in 40 mL of $THF/Et_3N$ (1/1) catalysts $Pd(PPh_3)_2Cl_2$ (0.124 g, 0.177 mmol, 0.05 equiv) and CuI (0.034 g, 0.177 mmol, 0.05 equiv) were added. After degazation the mixture was stirred at the room temperature for 5 min, then 15 mL of degazed solution of alkyne **46** (1.00 g, 3.55 mmol, 1.1 equiv) was added. The reaction mixture was degased with hydrogen, then stirred during 22 h at 50 °C in $Ar/H_2$ atmosphere. After concentration at reduced pressure the residue was purified by the flash chromatography ($CH_2Cl_2$/MeOH) to give 1.26g of 48 (90% yield). $^1H$ NMR (400 MHz, $CDCl_3$, δ): 8.42 (1H, dd, *J*= 8.4, 1.2 Hz), 7.97 (1H, d, *J* = 8.4 Hz), 7.59-7.55 (1H, m), 7.47-7.43 (1H, m), 7.27 (1H, d, *J* = 8.7 Hz), 7.21 (1H, d, *J*= 8.7 Hz), 3.95 (3H, s), 3.16 (2H, t, *J*= 6.3 Hz), 3.10 (2H, t, *J*= 6.3 Hz), 2.92 (2H, t, *J* = 7.3 Hz), 2.45 (2H, t, *J* = 6.9 Hz), 1.93-1.80 (4H, m), 1.76-1.69 (2H, m). $^{13}C$ NMR (100 MHz, $CDCl_3$, δ): 169.61, 159.18, 158.00, 146.50, 141.94, 136.14, 135.41, 133.21, 129.89, 129.07, 129.00, 126.64, 126.54, 126.07, 88.54, 80.42, 53.41, 35.08, 34.42, 29.38, 28.74, 23.19, 22.85, 18.71. HRMS (ESI) m/z: calculated for $C_{25}H_{25}N_2O_3S$ $[M+H]^+$ = 433.15858, found $[M+H]^+$ = 433.15887.

**Methyl 3-hydroxy-6-[5-(1,2,3,4-tetrahydroacridin-9-ylsulfanyl)pentyl]pyridine-2-carboxylate (49)**

To the degazed solution of compound **48** (0.62 g, 1.43 mmol, 1 equiv) in 30 mL of ethyl acetate $PtO_2$ (0.49 g, 2.15 mmol, 1.5 equiv) was added. The reaction mixture was stirred under $H_2$ at 5 bars during 21 h at rt. The solution

was filtered through celite, the solvent was evaporated, and the product was dried in *vacuum.* The crude product was purified by preparative TLC (c-hexane/ethyl acetate/$Et_3N$, 3/2/0.05) yielding 0.4 g of **49** (64%). $^1H$ NMR (400 MHz, $CDCl_3$, δ): 10.55 (1H, br. s.), 8.41 (1H, d, *J*= 8.2 Hz), 7.94 (1H, d, *J* = 8.2 Hz), 7.58 (1H, t, *J* = 7.6 Hz), 7.46 (1H, t, *J* = 7.6 Hz), 7.22 (1H, d, J = 8.7 Hz), 7.16 (1H, d, *J*= 8.7 Hz), 3.97 (3H, s), 3.15 (2H, t, *J*= 6.3 Hz), 3.10 (2H, t, *J*= 6.3 Hz), 2.73 (2H, t, *J*= 7.3 Hz), 2.68 (2H, t, *J*= 7.8 Hz), 1.94-1.82 (4H, m), 1.62-1.47 (4H, m), 1.42-1.34 (2H, m). $^{13}C$ NMR (100 MHz, $CDCl_3$, δ): 170.23, 159.08, 157.30, 153.84, 146.64, 142.08, 135.85, 129.21, 129.11, 129.01, 128.92, 128.78, 126.75, 126.23, 126.08, 53.28, 37.56, 36.01, 34.36, 30.13, 29.64, 29.28, 28.59, 23.22, 22.90. HRMS (ESI) m/z: calculated for $C_{25}H_{29}N_2O_3S$ $[M+H]^+$ 437.18988; found $[M+H]^+$ 437.19191.

**3-Hydroxy-6-[5-(1,2,3,4-tetrahydroacridin-9-ylsulfanyl)pentyl]pyridine-2-carbaldehyde (50)**

Compound **50** was obtained as an orange oil using ***general procedure 4*** (0.088 g, 42% (over three steps). $^1H$ NMR (400 MHz, $CDCl_3$, δ): 10.61 (1H, br. s.), 9.97 (1H, s), 8.43 (1H, d, *J* = 8.2 Hz), 7.96 (1H, d, J = 8.2 Hz), 7.60 (1H, m), 7.48 (1H, m), 7.23 (1H, d, *J* = 8.7 Hz), 7.18 (1H, d, *J* = 8.7 Hz), 3.17 (2H, t , *J* = 6.3 Hz), 3.12 (2H, t, *J* = 6.5 Hz), 2.78 (2H, t, *J* = 7.3 Hz), 2.69 (2H, t, *J* = 7.7 Hz), 1.97-1.84 (4H, m), 1.67-1.60 (2H, m, *J* = 7.7 Hz), 1.58-1.50 (2H, m, *J* = 7.3 Hz); 1.44-1.38 (2H, m). $^{13}C$ NMR (100 MHz, $CDCl_3$, δ): 198.89, 159.15, 157.14, 154.78, 146.67, 142.15, 135.91, 129.82, 129.17, 129.06, 128.85, 126.52, 126.30, 126.12, 37.27, 36.05, 34.39, 30.14, 29.33, 29.28, 28.54, 23.27, 22.94. HRMS (ESI) m/z: calculated for $C_{24}H_{27}N_2O_2S$ $[M+H]^+$ 407.17932, found for $[M+H]^+$ 407.17692.

**2-[1-(hydroxyimino)methyl]-6-[5-(1,2,3,4-tetrahydroacridin-9-ylsulfanyl)pentyl] pyridin-3-ol (14)**

Compound **14** was obtained accordingly to the ***general procedure 5*** using aldehyde **50** (0.143 g, 0.000352 mol, 1 equiv), $NH_2OH.HCl$ (0.037 g, 0.000528 mol, 1.5 equiv), $CH_3COONa$ (0.036 g, 0.000528 mol, 1.5 equiv), and 5 mL of ethanol. After evaporation of the solvent the crude product was purified by flash chromatography (c-hexane/ethyl acetate) to give compound **14** as a colorless solid (0.085 g, 57%). $^1H$ NMR (400 MHz, DMSO-d6, 8): 11.81 (1H, s), 10.07 (1H, s), 8.39 (1H, d, *J*= 8.4 Hz), 8.24 (1H, s), 7.89 (1H, d, *J* = 8.4 Hz), 7.68-7.64 (1H, m), 7.58-7.54 (1H, m), 7.23 (1H, d, *J* = 8.5 Hz), 7.05 (1H, d, *J* = 8.5 Hz), 3.12 (2H, t, *J* = 6.2 Hz) 3.02 (2H, t, *J* = 6.3 Hz), 2.82 (2H, t, *J* = 7.0 Hz), 2.56 (2H, t, *J* = 7.5 Hz), 1.90-1.79 (4H, m), 1.56-1.49 (2H, m), 1.47-1.40 (2H, m), 1.38-1.29 (2H, m). $^{13}C$ NMR (100 MHz, DMSO-d6, δ): 158.63, 152.59, 151.35, 151.07, 145.99, 140.62, 135.48, 135.27, 128.80, 128.60, 128.19, 126.20, 125.48, 123.95, 123.66, 36.17, 35.14, 33.55, 29.24, 28.67, 28.46, 27.54, 22.46, 22.09. HRMS (ESI) m/z: calculated for $C_{24}H_{28}N_3O_2S$ $[M+H]^+$ 422.19022; found $[M+H]^+$ 422.18876.

Example 2: Docking and molecular dynamics simulations

*Materials and Methods*

**Flexible docking conditions:**

Dockings have been performed using autodock vina (Ref http://onlinelibrary.wiley.com/doi/10.1002/jcc.21334/abstract) and by preparing the system in PyMOL (Schrödinger) using the plug-in developed by Daniel Seeliger (http://wwwuser.gwdg.de/-dseelig/adplugin.html). VX-hAChE was constructed from the apo form (pdb code 4EY4) by homology to the mAChE-VX structure (pdb code 2Y2U), keeping in the active site all the usually conserved water molecules. Residues in the gorge (Tyr72, Asp74, Trp86, Tyr124, Ser125, Trp286, Tyr337, Phe338, Tyr341) have been chosen as flexible, along with the ethyl group of VX. A docking box of 60x60x60 Angstroms was chosen, centered at the bottom of the gorge between Tyr124 and Trp86. Ligands were built and optimized from SMILEs string using Phenix elbow (Ref http://scripts.iucr.org/cgi-bin/paper?dz5186). The default parameter set of Autodock vina was used to generate 9 docking poses per molecule.

**MD simulations**

Molecular dynamics simulations were carried out using GROMACS 4.5.6 [Hess, B.; Kutzner, C.; van der Spoel, D.; Lindahl, E. J. Chem. Theory Comput. 2008, 4, 435-447.] and the Amber99sb force field. [Hornak, V.; Abel, R.; Okur, A.; Strockbine, B.; Roitberg, A.; Simmerling, C. Protein 2006, 65, 712-725]. The topological description of each KM molecule was built using acpype and the general amber force field [Wang, J.; Wolf, R. M.; Caldwell, J. W.; Kollman, P. A.; Case, D. A. J. Comput. Chem. 2004, 25, 1157-1174.]. The hAChE-VX complex in the conformation obtained from flexible dockings together with crystal water molecules and the different KM molecules, was immersed in a periodic water box of cubic shape with a minimal distance of 10 Å to any edge and periodic boundary conditions. The box was solvated using the TIP3P solvation model and chloride and sodium counter ions were added to neutralize the simulation

system. After energy minimization using a 500-step steepest decent method, the system was subjected to equilibration at 1 bar for 50 ps under the conditions of position restraints for heavy atoms. The solvent, the counter ions, and the protein were coupled separately to a temperature bath at 300 K. The Lennard-Jones interactions were cut off at 1.4 nm. The long-range electrostatic interactions were handled using particle-mesh Ewald method for determining long-range electrostatics (9 Å cutoff). Temperature was set to 300 K and was kept constant using a Berendsen thermostat [Berendsen, H. J. C.; Postma, J. P. M.; van Gunsteren, W. F.; DiNola, A.; Haak, J. R. J. Chem. Phys. 1984, 81, 3684] (with a coupling time constant of 0.1 ps). Pressure with a reference value of 1 bar was controlled by a Berendsen barostat (with a coupling time constant of 1 ps and a compressibility of 4.5 $10^5$ bar). Full MD simulation was performed for 5 ns at 300 K, using 2 femtosecond timesteps. All bond lengths were constrained using the LINCS algorithm allowing an integration step of 2 fs [Hess, B.; Bekker, H.; Berendsen, H. J. C.; Fraaije, J. G. E. M. J. Comput. Chem. 1997, 18, 1463]. Coordinates were saved every 250 steps *(i.e.* every 0.5 ps).

*Results*

Ternary complexes between VX-inhibited human acetylcholine esterase (hAChE) and 3-hydroxypyridine aldoxime **11** (5 carbons linker) and **12** (4 carbons linker) and derivatives with a shorter linker (3 and 2 carbons) have been studied by molecular docking and molecular dynamics simulations. Flexible docking experiments have been performed by allowing side-chain rotation of some key residues in the gorge starting from their native conformation. Each of the four docking results (Figure 1) indicated a conformational change of the peripheral-site Trp286 similar to the one seen in a bis-tacrine/*Tc*AChE complex (PDB code 2CEK). The tacrine moiety is found to form a $\pi$-$\pi$ stacking sandwich in between Tyr72 and Trp286. The scoring function yields similar binding energies for the 4 molecules (about - 9 kcal/mol), suggesting that the linker is sufficiently long to prevent unsuited accomodation of the molecule in the gorge.
The conformation resulting from the four dockings have then been equilibrated for 5 ns at a temperature of 300 K in molecular dynamics (MD) simulations. The distance between the aldoxime oxygen and the VX-AChE phosphorus atom was monitored along the simulation trajectory and the distribution of distances was calculated (Figure 2). A distance shorter than 4 Å means that the two atoms are sufficiently close to enhance the rate of formation of a covalent bond, i.e. that reactivation may occur at a fast rate. Molecule **12** brings the aldoxime oxygen both closer to the phosphorus atom and more frequently than the other three molecules, indicating that a 4-carbon linker is optimum for reactivation.

Example 3: Reactivation of hAChE inhibited by OPNAs

*Materials and methods*

**Inhibition of hAChE by OPNAs.** Recombinant hAChE was produced and purified as previously described (see reference: http://www.ncbi.nlm.nih.gov/pubmed/18975951) VX and tabun were from DGA maitrise NRBC (Vert le Petit, France). Paraoxon-ethyl was purchased from Sigma-Aldrich. HI-6 was from Pharmacie Centrale des Armées (Orléans, France). All other chemicals including paraoxon were from Sigma. Stock solution of VX and tabun were 5 mM in isopropanol. The inhibition of 120 $\mu$M hAChE was carried out with a 5-fold excess of OPNAs and was performed in tris buffer (20 mM, pH 7.4, 0.1% BSA) at 25 °C. After a 20-minute incubation, inhibited hAChE was desalted on PD-10 column (GE Healthcare).
**Reactivation of hAChE inhibited by OPNAs.** OPNA-inhibited hAChE was incubated at 37 °C with at least 5 concentrations of oxime in phosphate buffer (0.1 M, pH 7.4, 0.1% BSA). Every solution containing the different concentrations of oxime were transferred to 1-mL cuvettes at time intervals ranging from 1 to 10 minutes depending on the reactivation rate for measurement of hAChE activity using 1mM acetylthiocholine in Ellman's buffer (phosphate 0.1 M, pH 7.4, 0.1% BSA, 0.5 mM DTNB, 25 °C).
The enzyme activity in the control remained constant during the experiment. The percentage of reactivated enzyme (%$E_{react}$) was calculated as the ratio of the recovered enzyme activity and activity in the control. The apparent reactivation rate $k_{obs}$ for each oxime concentration, the dissociation constant $K_D$ of inhibited enzyme-oxime complex (E-POx) and the maximal reactivation rate constant $k_r$, were calculated by non-linear fit with ProFit (Quantumsoft) using the standard oxime concentration-dependent reactivation equation derived from the following scheme:

$$\text{E-P} + \text{Ox} \underset{}{\overset{K_D}{\rightleftharpoons}} \text{E-POx} \xrightarrow{k_r} \text{E} + \text{POx}$$

$$\%E_{react} = 100 \cdot (1-e^{k_{obs} \cdot t}) \quad \text{and} \quad k_{obs} = \frac{k_r[Ox]}{K_D + [Ox]}$$

*Results*

The ability of amidoximes **6, 8** and **9** and aldoximes **11** and **12** to reactivate *in vitro* VX, tabun and ethyl paraoxon-hAChE was studied by spectrophotometry using Ellman's reagent and compared to known reactivators such as 2-PAM, obidoxime, HLö-7, trimedoxime (TMB4) and HI-6.
The results are provided in tables 1 and 2 below.

Table 1. *In vitro* Reactivation of VX-hAChE

| Reactivator | $k_r$ (min$^{-1}$) | $K_D$ ($\mu$M) | $k_{r2}$ (mM$^{-1}$min$^{-1}$)[b] |
|---|---|---|---|
| 2-PAM | 0.06±0.01 | 215±75 | 0.28 |
| Obidoxime | 0.60±0.05 | 54±12 | 11 |
| HLö-7 | 0.49[a] | 7.8[a] | 63[a] |
| Trimedoxime | nd[c] | nd[c] | 0.50±0.02[c] |
| HI-6 | 0.44±0.15 | 50±26 | 9 |
| **6** | nd[c] | nd[c] | 0.65[c] |
| **8** | 0.0094±0.0004 | 15±2 | 0.6 |
| **9** | 0.032±0.001 | 30±3 | 1.1 |
| **11** | 0.56±0.12 | 41±11 | 13 |
| **12** | 0.72±0.07 | 31±6 | 22 |

[a] from the ref. [33] [b] $k_{r2}= k_r/K_D$ [c] if [reactivator] << $K_D$, then there is a linear dependence between $k_{obs}$ and [reactivator]: $k_{obs}$ = ($kr/K_D$)[reactivator]. In this case, $k_r$ and $K_D$ cannot be determined, but $k_{r2} = k_r/K_D$ is the slope of the line. [d] Not determined if $k_{obs}$ is < 0.01 min$^{-1}$ at practical concentration.

Table 2. *In vitro* reactivation of tabun-hAChE

| Reactivator | $k_r$ (min$^{-1}$) | $K_D$ ($\mu$M) | $k_{r2}$ (mM$^{-1}$min$^{-1}$)[b] |
|---|---|---|---|
| Obidoxime | 0.04±0.006 | 250±110 | 0.16 |
| HLö-7 | 0.020±0.0007 | 106±15 | 0.2 |
| Trimedoxime | 0.085±0.005 | 145±25 | 0.7 |
| **11** | - | - | 0.06±0.007 |
| **12** | 0.021±0.001 | 7.1±1.5 | 3 |

In contrast, 4-substituted tetrahydroacridine $\alpha$-hydroxypyridine amidoximes **6, 8** and **9** bearing C5 alkyl and *N*-Me containing linkers, respectively, due to the improved affinity exhibited higher reactivation efficiencies ($k_{r2}$) for VX-AChE compared to 2-PAM and trimedoxime, but they were less efficient than obidoxime, HLö-7,and HI-6. Yet, it is important to notice that these compounds are the first described efficient amidoxime-based reactivators for VX-inhibited AChE. Remarkably, aldoximes **11** and **12** substituted in position 6 of the pyridine ring by the C5 and C4 alkyl linker were more efficient in reactivating VX-hAChE than 2-PAM, obidoxime, trimedoxime, and HI-6. Although oximes **11** and **12** do not overpass the reactivating efficiency of HLö-7,they display another interesting starting point for further improvement, since they do not bear neither a permanent charge nor a tertiary amine which could hamper the BBB crossing ability.
It is important to point out that oxime **12** reactivates effectively tabun- hAChE (Table 2), which is known to be reluctant to reactivation, due to weak electrophilicity and steric hindrance of the tabun-hAChE adduct. Compound **12** exhibited much higher *in vitro* reactivation potency compared to all current quaternary pyridinium aldoximes. Despite a lower

reactivation rate constant ($k_r$) compared to obidoxime and trimedoxime, **12** due to the significantly improved affinity toward inhibited enzyme ($K_D$) was 4-fold more efficient than trimedoxime, the best pyridinium aldoxime reactivator of tabun- hAChE. The better reactivity of oxime **12** on VX- and tabun-hAChE compared to **11** suggests that the shorter linker allows better binding to the inhibited enzyme as well as more accurate orientation of the oxime function for displacement of the phosphyl or phosphoramidyl moiety.

Most organophosphate pesticide poisoning results from the formation of a diethylphosphoryl conjugate of hAChE. Testing molecules for their ability to reactivate ethylparaoxon-inhibited hAChE, which results in the formation of the diethylphosphoryl conjugate, provides clues about their general efficacy at countering pesticide poisoning. It appears that compounds **11** and **12** are more efficient than obidoxime for the reactivation of paraoxon-hAChE, and superior to HLö-7 (Table 3). Much like the trend observed in the case of VX, decreasing the linker length from 5 to 4 carbons leads to a 3-fold increase in $K_D$ balanced by a 5-fold increase in *k*.

Table 3. *In vitro* reactivation of paraoxon-hAChE

| Reactivator | $k_r$ ($min^{-1}$) | $K_D$ ($\mu M$) | $k_{r2}$ ($mM^{-1}min^{-1}$) [b] |
|---|---|---|---|
| Obidoxime | 0.81[a] | 32.2[a] | 20[a] |
| HLö-7 | 0.63±0.04 | 210±31 | 3 |
| Trimedoxime | 0.34±0.02[a] | 47.8±6.9[a] | 17[a] |
| **11** | 0.0228±0.0006 | 1.1±0.1 | 20.9 |
| **12** | 0.111±0.002 | 3.6±0.2 | 31 |
| [a] from the ref. [34] [a] $k_{r2} = k_r/K_D$. | | | |

<u>Example 4: Inhibition of hAChE with compounds of the invention</u>

Compounds **7, 10, 11** and **12** were found to inhibit native hAChE with $IC_{50}$ similar to tacrine and tacrine-pyridyl heterodimer **51** (Table 4).

Table 4. Inhibition of hAChE

| compound | $IC_{50}$ ($\mu M$) | compound | $IC_{50}$ ($\mu M$) |
|---|---|---|---|
| Tacrine | 0.205±18 (human erythrocyte AChE)[a] | **12** | 1.40±0.03 (hAChE) |
| 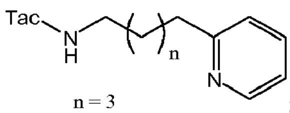 n = 3 **51** | 0.15±0.01 (hAChE) | **13** | 0.163 (hAChE) |
| 7 | 0.26+0.01 (hAChE) | | |
| **10** | 0.077±0.003 (hAChE) | | |
| **11** | 0.26±0.01 (hAChE) | | |

Interestingly, 4-substituted pyridine aldoxime **10,** contrary to 6-substituted compound **11** showed no reactivation of VX-, tabun- and paraoxon inhibited hAChE and a higher inhibition compared to tacrine. This suggests that 4-substitution of pyridine ring favors the binding of tacrine moiety to the catalytic site of the enzyme by contrast to the peripheral site.

## Claims

**1.** Compound of formula (I)

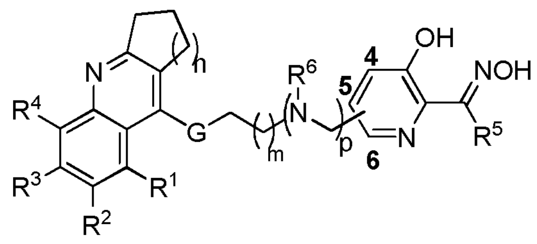

(II) ,

wherein

G is selected from the group consisting of $CH_2$, O, S, NH, NR, C(O)-NH, C(O)-NR, NH-C(O), NR-C(O), NH-C(S), NR-C(S), NH-NR, NR-NR', NH-O, NR-O, NH-C-(O)-NH, NR-C(O)-NH, NR-C(O)-NR', NH-C(S)-NH, NR-C(S)-NH, and NR-C(S)-NR',
wherein each R or R' is independently selected from the group consisting of a hydrogen atom, an alkyl group, an acyl group, an aryl group and a heteroaryl group;
n is 1, 2, 3 or 4,
m is 0, 1,2, 3, or 4,
p is 0 or 1,
$R^1$, $R^2$, $R^3$ and $R^4$ are independently selected from the group consisting of a hydrogen atom, a halogen atom, an alkyl group, an alkoxy group, an amine NHR group, an amide NR''-C(O)-R''' group, an oligopeptide, or a polyethyleneglycol chain, wherein R'' and
R''' have the same definition as R and R',
$R^5$ is a hydrogen atom, a trifluoromethyl group, or a $NH_2$ group, and
$R^6$ is a hydrogen atom, an alkyl group, an aryl group or an acyl group.

**2.** Compound according to claim 1, wherein m is 3.

**3.** Compound according to anyone of claims 1 and 2, wherein n is 2.

**4.** Compound according to anyone of claims 1 to 3, wherein p is 0.

**5.** Compound according to anyone of claims 1 to 4, wherein the pyridine is substituted in position 6.

**6.** Compound according to anyone of claims 1 to 5, wherein $R^5$ is a hydrogen atom.

**7.** Compound according to anyone of claims 1 to 6, wherein three among $R^1$, $R^2$, $R^3$ and $R^4$ are hydrogen atoms.

**8.** Compound according to claim 7, wherein $R^1$, $R^2$, $R^3$ and $R^4$ are hydrogen atoms.

**9.** Compound according to anyone of claims 1 to 7, wherein G is NH.

**10.** Compound according to anyone of claims 1 to 3 and 5 to 9, wherein $R^6$ is an alkyl group, preferably a methyl group.

**11.** Compound according to anyone of claims 1 to 10, wherein the compound is selected from the group consisting of:

N',3-dihydroxy-4-{5-[(1,2,3,4-tetrahydroacridin-9-yl)amino]pentyl}pyridine-2-carboximidamide **(6)**
N',3-dihydroxy-6-{5-[(1,2,3,4-tetrahydroacridin-9-yl)amino]pentyl}pyridine-2-carboximidamide (7)
(Z)-N',3-dihydroxy-4-{[methyl({2-[(1,2,3,4-tetrahydroacridin-9-yl)amino]ethyl})amino]methyl}pyridine-2-carboximidamide **(8)**
(Z)-N',3-dihydroxy-4-{[methyl({3-[(1,2,3,4-tetrahydroacridin-9-yl)amino]propyl})amino]methyl}pyridine-2-carboximidamide **(9),**
2-[(hydroxyimino)methyl]-4-{5-[(1,2,3,4-tetrahydroacridin-9-yl)amino]pentyl} pyridin-3-ol **(10),**
2-[(1E)-(hydroxyimino)methyl]-6-{5-[(1,2,3,4-tetrahydroacridin-9-yl)amino]pentyl} pyridin-3-ol **(11)**
2-[1-(hydroxyimino)methyl]-6-{4-[(1,2,3,4-tetrahydroacridin-9-yl)amino]butyl} pyridin-3-ol **(12),**
2-[1-(hydroxyimino)methyl]-5-{5-[(1,2,3,4-tetrahydroacridin-9-yl)amino]pentyl} pyridin-3-ol **(13),** and

2-[1-(hydroxyimino)methyl]-6-[5-(1,2,3,4-tetrahydroacridin-9-ylsulfanyl)pentyl] pyridin-3-ol **(14).**

**12.** Pharmaceutical composition comprising at least one compound of formula (I) as defined in anyone of claims 1 to 11 and at least one pharmaceutically acceptable support.

**13.** Compound according to anyone of claims 1 to 11, for use as a medicament.

**14.** Compound according to anyone of claims 1 to 11, *for in vitro* use as a reactivator of human acetylcholinesterase, wherein the human acetylcholinesterase was inhibited by at least one organophosphorus nerve agents.

**15.** Compound according to anyone of claims 1 to 11, for use in the treatment of a nervous and/or respiratory failure due to intoxication with at least one organophosphorus nerve agent.

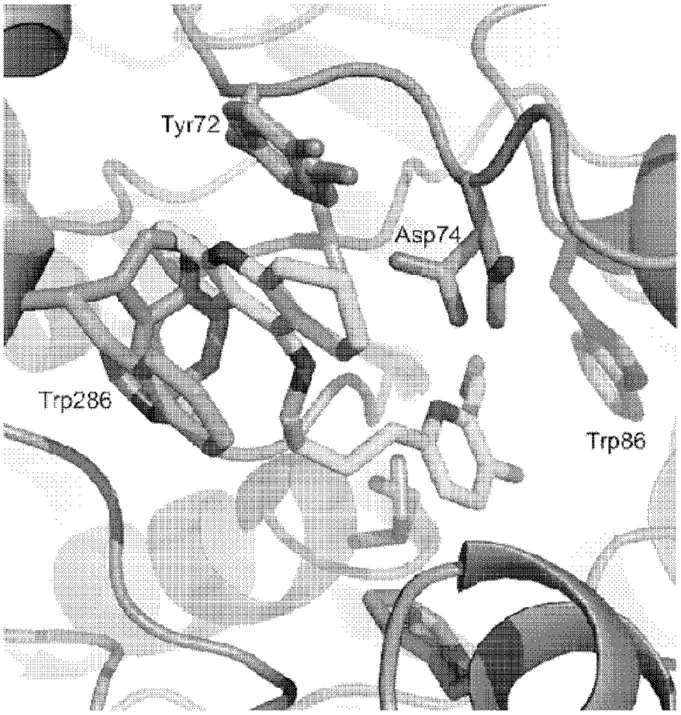


Figure 1

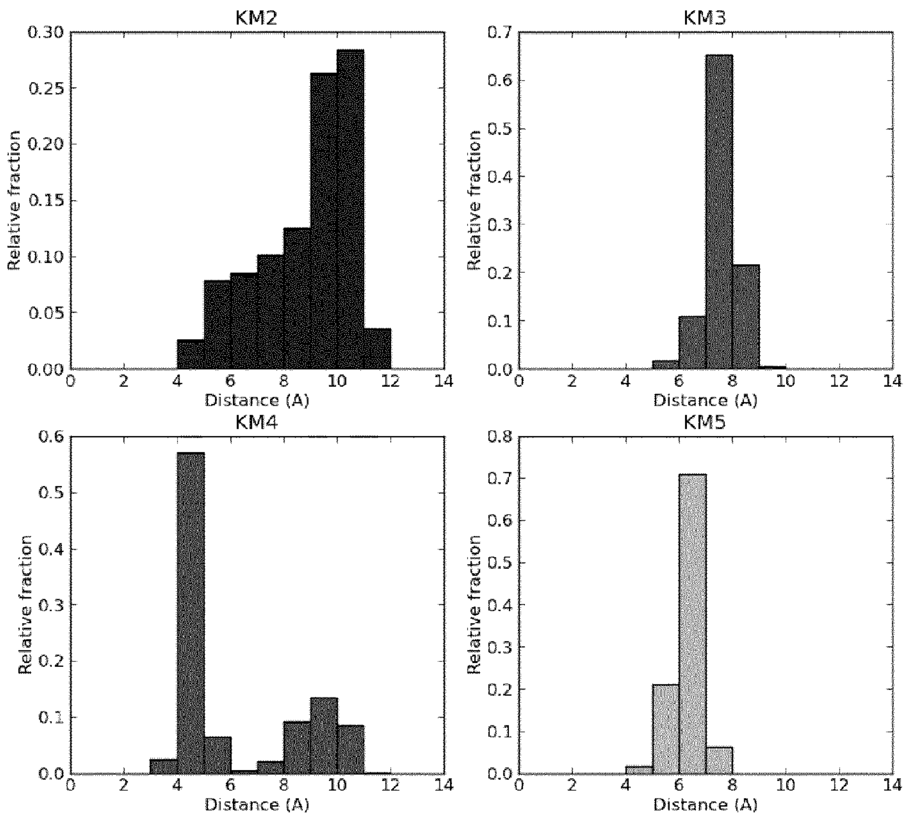


Figure 2

Europäisches Patentamt
European Patent Office
Office européen des brevets

# EUROPEAN SEARCH REPORT

Application Number
EP 13 30 6584

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GUILLAUME MERCEY ET AL: "Phenyltetrahydroisoquinoline-Pyridinaldoxime Conjugates as Efficient Uncharged Reactivators for the Dephosphylation of Inhibited Human Acetylcholinesterase", JOURNAL OF MEDICINAL CHEMISTRY, vol. 55, no. 23, 13 December 2012 (2012-12-13), pages 10791-10795, XP055099217, ISSN: 0022-2623, DOI: 10.1021/jm3015519 * the whole document * ----- | 1-15 | INV. C07D401/12 A61K31/473 A61P25/00 |
| A | FEI MAO ET AL: "O-Hydroxyl- or o-amino benzylamine-tacrine hybrids: Multifunctional biometals chelators, antioxidants, and inhibitors of cholinesterase activity and amyloid-[beta] aggregation", BIOORGANIC & MEDICINAL CHEMISTRY, vol. 20, no. 19, 1 October 2012 (2012-10-01), pages 5884-5892, XP055099195, ISSN: 0968-0896, DOI: 10.1016/j.bmc.2012.07.045 * table 1 * ----- | 1-15 | TECHNICAL FIELDS SEARCHED (IPC): C07D |
| A | PAWE SZYMASKI ET AL: "Synthesis and biological activity of derivatives of tetrahydroacridine as acetylcholinesterase inhibitors", BIOORGANIC CHEMISTRY, vol. 39, no. 4, 2011, - 2011, pages 138-142, XP028296972, ISSN: 0045-2068, DOI: 10.1016/J.BIOORG.2011.05.001 [retrieved on 2011-05-12] * table 2 * ----- -/-- | 1-15 | |

The present search report has been drawn up for all claims

2

| Place of search | Date of completion of the search | Examiner |
|---|---|---|
| Munich | 30 January 2014 | Fanni, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 30 6584

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CARLIER P R ET AL: "Heterodimeric tacrine-based acetylcholinesterase inhibitors: investigating ligand-peripheral site interactions", JOURNAL OF MEDICINAL CHEMISTRY, AMERICAN CHEMICAL SOCIETY, US, vol. 42, no. 20, 7 October 1999 (1999-10-07), pages 4225-4231, XP002434602, ISSN: 0022-2623, DOI: 10.1021/JM990224W * table 2 * ----- | 1-15 | |

TECHNICAL FIELDS SEARCHED (IPC)

The present search report has been drawn up for all claims

2

| Place of search | Date of completion of the search | Examiner |
|---|---|---|
| Munich | 30 January 2014 | Fanni, Stefano |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HESS, B. ; KUTZNER, C. ; VAN DER SPOEL, D. ; LINDAHL, E. J.** *Chem. Theory Comput.,* 2008, vol. 4, 435-447
- **HORNAK, V. ; ABEL, R. ; OKUR, A. ; STROCKBINE, B. ; ROITBERG, A. ; SIMMERLING, C.** *Protein,* 2006, vol. 65, 712-725
- **WANG, J. ; WOLF, R. M. ; CALDWELL, J. W. ; KOLLMAN, P. A. ; CASE, D. A.** *J. Comput. Chem.,* 2004, vol. 25, 1157-1174
- **BERENDSEN, H. J. C. ; POSTMA, J. P. M. ; VAN GUNSTEREN, W. F. ; DINOLA, A. ; HAAK, J. R.** *J. Chem. Phys.,* 1984, vol. 81, 3684
- **HESS, B. ; BEKKER, H. ; BERENDSEN, H. J. C. ; FRAAIJE, J. G. E. M.** *J. Comput. Chem.,* 1997, vol. 18, 1463